# EUROPEAN PATENT APPLICATION

(11) **EP 1 614 693 A1**
(43) Date of publication of application: **11.01.2006**
(21) Application number: 04720750.1
(22) Date of filing: 15.03.2004
(51) Int. Cl.: C07K 16/18, C07K 1/18, C07K 1/20, C07K 1/22, C07K 1/36

(54) **PURIFICATION OF HUMAN MONOCLONAL ANTIBODY AND HUMAN POLYCLONAL ANTIBODY**

(30) Priority: 31.03.2003 JP 2003097407
(71) Applicant: KIRIN BEER KABUSHIKI KAISHA, Tokyo 104-8288 (JP)
(72) Inventor: ISHIHARA, Takashi, Takasaki Pharmaceutical Plant,, Takasaki-shi Gunma, 3700013 (JP)
(74) Representative: HOFFMANN EITLE
(86) International application number: PCT/JP2004/003425
(87) International publication number: WO 2004/087761

(57) **Abstract**

This invention provides a method for separating and purifying human monoclonal antibodies and human polyclonal antibodies from an antibody-containing mixture where such antibodies are primarily used for therapeutic applications and are occasionally used for diagnostic and reagent applications. This method comprises at least the following steps of: (1) purification via anion exchange chromatography; (2) purification via cation exchange chromatography following step (1); and separation of human antibodies from artiodactyl antibodies from a mixture that contains human antibodies and artiodactyl antibodies via protein A affinity chromatography that employs pH gradient elution.

## Description

### Technical Field

The present invention relates to purification of antibodies. More particularly, the present invention relates to a method for separating and purifying human monoclonal antibodies and human polyclonal antibodies where such antibodies are primarily used for therapeutic applications and occasionally used for diagnostic and reagent applications.

### Background Art

### (1) Purification of monoclonal antibodies

In general, antibody purification involves the use of a protein A column. In the case of antibody drugs that have drawn attention in recent years, such as monoclonal antibody drugs, antibodies of high purity are required for therapeutic applications. Accordingly, a process of purifying an antibody drug is generally carried out via conventional chromatography, such as gel filtration, ion exchange, or hydrophobic chromatography, in addition to affinity chromatography on a protein A column.

For example, Rhona M. O'Leary et al. conducted purification of antibodies for therapy via a sequence of affinity chromatography on a protein A column and cation exchange chromatography, followed by anion exchange chromatography (BioPharm, September 2001, 10-18). In such purification method, however, the combination of cation exchange column chromatography followed by anion exchange column chromatography is problematic. More specifically, this process requires a procedure of dilution (resulting in conductivity of 5 mS/cm or lower in general) for decreasing the ion intensity of an antibody eluate before the antibody eluate resulting from cation exchange chromatography is subjected to anion exchange chromatography. As a result, this process of dilution causes problems in a sequence of purification processes. For example, (i) a solution for dilution becomes necessary, (ii) the size of an apparatus used is enlarged because of an increased volume resulting from dilution, and (iii) operating time is prolonged. The process of dilution is necessary for the following reasons. While antibodies are generally eluted by increasing the ion intensity via cation exchange chromatography, antibodies are generally allowed to pass through the anion column by decreasing the ion intensity, and impurities are allowed to adsorb on the anion column via a next step of anion exchange chromatography.

### (2) Purification of human antibodies from nonhuman animal polyclonal antibodies

A variety of techniques have been developed as methods for producing human antibodies. For example, DNA encoding human antibodies may be introduced into animal cells to produce the human antibodies in such animal cells. In such a case, serum derived from a nonhuman animal is usually added when culturing the animal cells. Thus, the culture supernatant of the human antibody-containing animal cells contains the nonhuman animal antibodies.

Also, a transgenic nonhuman animal into which the locus of a human antibody has been introduced has been produced. Immunization of such animal with an antigen can result in the production of a human antibody against the aforementioned antigen in the animal. However, the body fluids of the immunized animal, such as emulsions or blood, contain the antibody of the animal itself as well as a human antibody.

When separating and purifying human antibodies from human antibody-producing cells, the human antibodies must be separated from a medium that contains a serum component including nonhuman animal-derived antibodies. When separating and purifying human antibodies from the body fluids, such as emulsions or blood, of a transgenic nonhuman animal capable of producing human antibodies, the human antibodies must be separated from the body fluids containing antibodies derived from the transgenic nonhuman animal.

For example, mouse monoclonal antibodies are separated from bovine serum-derived bovine IgG antibodies via hydrophobic chromatography (Grunfeld H. et al., J. Immunol. Methods 1997, 201 (2)). An example of separation of a mouse monoclonal antibody and bovine serum-derived bovine IgG antibody is protein A affinity chromatography that employs pH gradient elution (Antibody Purification Handbook, Amersham Biosciences, 72-0823-01, p. 88). Also, there is a report that a monoclonal antibody produced from a transgenic goat is separated and purified via protein A affinity chromatography, protein G affinity chromatography, ion-exchange chromatography, or hydrophobic chromatography, which has resulted in a greater than 3 log 10 reduction in the level of goat-derived IgG (Daniel P. Pollock et al., J. Immunological Methods, 231, 1999).

However, there has been no report on the separation of a human antibody from a bovine or sheep antibody. Further, there has been no report on the separation of a bovine, goat; or sheep polyclonal antibody from a human polyclonal antibody.

### Disclosure of the Invention

(1) In antibody purification, the step of dilution that is essential in conventional procedures of "cation exchange chromatography followed by anion exchange chromatography" has been problematic in terms of, for example, (i) necessity of a solution for dilution, (ii) necessity of an enlarged apparatus due to an increased volume resulting from dilution, and (iii) prolonged operating time. (2) A method for isolating a human antibody from a mixture containing an ungulate antibody and a human antibody has not yet been developed. Also, a method for isolating a human polyclonal antibody from a mixture containing a human polyclonal antibody and an ungulate polyclonal antibody has not yet been developed.

The present invention is directed to solving the aforementioned problems.

The present inventor has conducted concentrated studies in order to effectively purify antibodies. As a result, I found that a purification sequence of anion exchange chromatography followed by cation exchange chromatography would solve the aforementioned problems, thereby completing the present invention. Separately, I have conducted concentrated studies in order to find a method for isolating a human antibody from a mixture comprising a human antibody and an ungulate antibody such as a bovine, sheep, or goat antibody. As a result, I found that a human antibody could be isolated by subjecting such mixture to a protein A column and then performing pH-gradient elution. This has led to the completion of the present invention.

Specifically, the present invention is as follows.
1. A method for separating and purifying antibodies from an antibody-containing mixture comprising at least the following steps of:
   (1) purification via anion exchange chromatography; and
   (2) purification via cation exchange chromatography following step (1).
2. A method for separating and purifying antibodies from an antibody-containing mixture comprising at least the following steps of:
   (1) purification via protein A affinity chromatography;
   (2) purification via anion exchange chromatography following step (1); and
   (3) purification via cation exchange chromatography following step (2).
3. A method for separating and purifying antibodies from an antibody-containing mixture comprising at least the following steps of:
   (1) purification via protein A affinity chromatography;
   (2) purification via anion exchange chromatography following step (1);
   (3) purification via cation exchange chromatography following step (2); and
   (4) purification via hydrophobic chromatography following step (3).
4. A method for separating and purifying antibodies from an antibody-containing mixture comprising at least the following steps of:
   (1) applying an antibody-containing mixture to a protein A column equilibrated with a buffer containing sodium phosphate and sodium chloride to bring the mixture into contact with the protein A column;
   (2) washing the protein A column with the buffer used for equilibration in step (1) following step (1);
   (3) eluting the antibodies with a sodium citrate buffer following step (2);
   (4) adjusting the pH of an eluate to 4.0 to 8.5 with a sodium phosphate buffer following step (3);
   (5) adjusting the pH of the eluate of step (4) to 6.0 to 8.5 with a Tris-HCl buffer following step (4);
   (6) applying the eluate of step (5) to an anion exchange column equilibrated with a Tris-HCl buffer to bring the eluate into contact with the anion exchange column following step (5);
   (7) eluting the antibodies from the anion exchange column with the buffer used for equilibration in step (6) following step (6);
   (8) adjusting the pH of the eluate of step (7) to 4.0 to 7.0 with acetic acid or citric acid following step (7);
   (9) applying the eluate of step (8) to a cation exchange column equilibrated with a sodium acetate buffer or a sodium phosphate-citrate buffer to bring the eluate into contact with the cation exchange column following step (8);
   (10) washing the cation exchange column with the buffer used for equilibration in step (9) following step (9); and
   (11) eluting the antibodies from the cation exchange column with a sodium acetate buffer or a sodium phosphate-citrate buffer (buffer containing sodium phosphate and citrate) and a buffer containing sodium chloride following step (10).
5. A method for separating and purifying antibodies from an antibody-containing mixture, which further comprises the steps of:
   (12) applying ammonium sulfate to the eluate of step (11) of 4 to adjust the pH of the eluate to 6.0 to 8.0 with an aqueous solution of sodium hydroxide following step (11) of 4;
   (13) applying the eluate of step (12) to a hydrophobic column equilibrated with a buffer containing ammonium sulfate and sodium phosphate to bring the eluate into contact with the hydrophobic column following step (12);
   (14) washing the hydrophobic column with the buffer used for equilibration in step (13) following step (13); and
   (15) eluting the antibodies from the hydrophobic column by lowering the ammonium sulfate concentration in the buffer of step (13) following step (14).
6. The method according to any one of 1 to 5, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
7. The method according to 6, wherein the step of virus inactivation is carried out following the step of purification via protein A chromatography.
8. The method according to 7, wherein the step of virus inactivation is carried out following step (3) of 4 by adjusting the pH of the eluate obtained in step (3) to 4 or lower and allowing the eluate to stand for 30 minutes or longer.
9. The method according to any one of 6 to 8, wherein the step of virus removal via filtration with the use of a virus removal filter and/or the step of aseptic filtration are carried out in the final step of the method for separating and purifying antibodies from an antibody-containing mixture.
10. The method according to any one of 4 to 9, wherein a Tris-HCl buffer is used instead of the sodium phosphate buffer of step (4) of 4.
11. The method according to any one of 4 to 9, wherein the following step is carried out instead of step (4) and step (5) of 4:
   a step of adjusting the pH of an eluate to 6.0 to 8.5 with a Tris-HCl buffer following step (3).
12. A method for separating and purifying antibodies from an antibody-containing mixture comprising at least the following steps of:
   (1) applying an antibody-containing mixture to a protein A column equilibrated with a buffer containing 10 mM to 100 mM sodium phosphate and 0 M to 4.0 M sodium chloride to bring the mixture into contact with the protein A column;
   (2) washing the protein A column with the buffer used for equilibration in step (1) following step (1);
   (3) eluting the antibodies with a 10 mM to 100 mM sodium citrate buffer following step (2);
   (4) adjusting the pH of an eluate to 4.0 to 8.5 with a 10 mM to 200 mM sodium phosphate buffer following step (3);
   (5) adjusting the pH of the eluate of step (4) to 6.0 to 8.5 with a 0.5 M to 1.5 M Tris-HCl buffer following step (4);
   (6) applying the eluate of step (5) to an anion exchange column equilibrated with a 10 mM to 20 mM Tris-HCl buffer to bring the eluate into contact with the anion exchange column following step (5);
   (7) eluting the antibodies from the anion exchange column with the buffer used for equilibration in step (6) following step (6);
   (8) adjusting the pH of the eluate of step (7) to 4.0 to 7.0 with 0.5 M to 1.5 M acetic acid or citric acid following step (7);
   (9) applying the eluate of step (8) to a cation exchange column equilibrated with a 10 mM to 50 mM sodium acetate buffer or a 10 mM to 50 mM sodium phosphate-10 mM to 50 mM citrate buffer to bring the eluate into contact with the cation exchange column following step (8);
   (10) washing the cation exchange column with the buffer used for equilibration in step (9) following step (9); and
   (11) eluting the antibodies from the cation exchange column with a 10 mM to 50 mM sodium acetate or 10 mM to 50 mM sodium phosphate-10 mM to 50 mM citrate buffer and a buffer containing 0.1 M to 1.0 M sodium chloride following step (10).
13. A method for separating and purifying antibodies from an antibody-containing mixture, which further comprises the steps of:
   (12) applying ammonium sulfate to the eluate of step (11) of 12 to bring the ammonium sulfate concentration thereof to 0.8 M to 1.2 M and adjusting the pH thereof to 6.0 to 8.0 with an aqueous solution of 1.0 M to 10.0 M sodium hydroxide following step (11) of 12;
   (13) applying the eluate of step (12) to a hydrophobic column equilibrated with a buffer containing 0.8 M to 1.2 M ammonium sulfate and 10 mM to 100 mM sodium phosphate to bring the eluate into contact with the hydrophobic column following step (12);
   (14) washing the hydrophobic column with the buffer used for equilibration in step (13) following step (13); and
   (15) eluting the antibodies from the hydrophobic column by lowering the ammonium sulfate concentration in the buffer of step (13) following step (14).
14. The method according to 12 or 13, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
15. The method according to 14, wherein the step of virus inactivation is carried out following step (3) of 12 by adjusting the pH of the eluate obtained in step (3) to 4 or lower and allowing the eluate to stand for 30 minutes or longer.
16. The method according to 14 or 15, wherein the step of virus removal via filtration with the use of a virus removal filter and/or the step of aseptic filtration are carried out following step (11) of 12.
17. The method according to 14 or 15, wherein the step of virus removal via filtration with the use of a virus removal filter and/or the step of aseptic filtration are carried out following step (15) of 13.
18. The method according to any one of 12 to 17, wherein a 0.5 M to 1.5 M Tris-HCl buffer is used instead of the sodium phosphate buffer of step (4) of 12.
19. The method according to any one of 12 to 17, wherein the following step is carried out instead of step (4) and step (5) of 12:
   a step of adjusting the pH of an eluate to 6.0 to 8.5 with a 0.5 M to 1.5 M Tris-HCl buffer following step (3).
20. A method for separating and purifying antibodies from an antibody-containing mixture comprising at least the following steps of:
   (1) applying an antibody-containing mixture to a protein A column equilibrated with a buffer containing 10 mM to 20 mM sodium phosphate and 0.15 M sodium chloride to bring the mixture into contact with the protein A column;
   (2) washing the protein A column with the buffer used for equilibration in step (1) following step (1);
   (3) eluting the antibodies with a 10 mM to 20 mM sodium citrate buffer following step (2);
   (4) adjusting the pH of an eluate to 4.0 to 8.5 with a 10 mM to 100 mM sodium phosphate buffer following step (3);
   (5) adjusting the pH of the eluate of step (4) to 6.0 to 8.5 with a 1.0 M to 1.5 M Tris-HCl buffer following step (4);
   (6) applying the eluate of step (5) to an anion exchange column equilibrated with a 10 mM Tris-HCl buffer to bring the eluate into contact with the anion exchange column following step (5);
   (7) eluting the antibodies from the anion exchange column with the buffer used for equilibration in step (6) following step (6);
   (8) adjusting the pH of the eluate of step (7) to 4.0 to 7.0 with 1 M acetic acid or citric acid following step (7);
   (9) applying the eluate of step (8) to a cation exchange column equilibrated with a 20 mM sodium acetate buffer or a 20 mM sodium phosphate-10 mM citrate buffer to bring the eluate into contact with the cation exchange column following step (8);
   (10) washing the cation exchange column with the buffer used for equilibration in step (9) following step (9); and
   (11) eluting the antibodies from the cation exchange column with a 20 mM sodium acetate or 20 mM sodium phosphate-10 mM citrate buffer and a buffer containing 0.3 M sodium chloride following step (10).
21. A method for separating and purifying antibodies from an antibody-containing mixture, which further comprises the steps of:
   (12) applying ammonium sulfate to the eluate of step (11) of 20 to bring the ammonium sulfate concentration thereof to 1.0 M and adjusting the pH thereof to 6.0 to 8.0 with an aqueous solution of 10.0 M sodium hydroxide following step (11) of 20;
   (13) applying the eluate of step (12) to a hydrophobic column equilibrated with a buffer containing 1.0 M ammonium sulfate and 10 mM sodium phosphate to bring the eluate into contact with the hydrophobic column following step (12);
   (14) washing the hydrophobic column with the buffer used for equilibration in step (13) following step (13); and
   (15) eluting the antibodies from the hydrophobic column by lowering the ammonium sulfate concentration in the buffer of step (13) following step (14).
22. The method according to 20 or 21, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
23. The method according to 22, wherein the step of virus inactivation is carried out following step (3) of 20 by adjusting the pH of the eluate obtained in step (3) to 4 or lower and allowing the eluate to stand for 30 minutes or longer.
24. The method according to 22 or 23, wherein the step of virus removal via filtration with the use of a virus removal filter and/or the step of aseptic filtration are carried out following step (11) of 20.
25. The method according to 22 or 23, wherein the step of virus removal via filtration with the use of a virus removal filter and/or the step of aseptic filtration are carried out following step (15) of 21.
26. The method according to any one of 20 to 25, wherein a 1.0 M Tris-HCl buffer is used instead of the sodium phosphate buffer of step (4) of 20.
27. The method according to any one of 20 to 25, wherein the following step is carried out instead of step (4) and step (5) of 20:
   a step of adjusting the pH of an eluate to 6.0 to 8.5 with a 1.0 M Tris-HCl buffer following step (3).
28. A method for separating and purifying antibodies from an antibody-containing mixture comprising at least the following steps of:
   (1) applying an antibody-containing mixture to a protein A column equilibrated with a buffer containing 10 mM to 20 mM sodium phosphate and 0.15 M sodium chloride to bring the mixture into contact with the protein A column;
   (2) washing the protein A column with the buffer used for equilibration in step (1) following step (1);
   (3) eluting the antibodies with a 10 mM to 20 mM sodium citrate buffer following step (2);
   (4) adjusting the pH of an eluate to 7.0 with a 10 mM to 100 mM sodium phosphate buffer following step (3);
   (5) adjusting the pH of the eluate of step (4) to 8.0 with a 1.0 M to 1.5 M Tris-HCl buffer following step (4);
   (6) applying the eluate of step (5) to an anion exchange column equilibrated with a 10 mM Tris-HCl buffer to bring the eluate into contact with the anion exchange column following step (5);
   (7) eluting the antibodies from the anion exchange column with the buffer used for equilibration in step (6) following step (6);
   (8) adjusting the pH of the eluate of step (7) to 5.0 with 1.0 M acetic acid or citric acid following step (7);
   (9) applying the eluate of step (8) to a cation exchange column equilibrated with a 20 mM sodium acetate buffer or a 20 mM sodium phosphate-10 mM citrate buffer to bring the eluate into contact with the cation exchange column following step (8);
   (10) washing the cation exchange column with the buffer used for equilibration in step (9) following step (9); and
   (11) eluting the antibodies from the cation exchange column with a 20 mM sodium acetate or 20 mM sodium phosphate-10 mM citrate buffer and a buffer containing 0.3 M sodium chloride following step (10).
29. A method for separating and purifying antibodies from an antibody-containing mixture, which further comprises the steps of:
   (12) adding ammonium sulfate to the eluate of step (11) of 28 to bring the ammonium sulfate concentration thereof to 1.0 M and adjusting the pH thereof to 7.0 with an aqueous solution of 10.0 M sodium hydroxide following step (11) of 28;
   (13) applying the eluate of step (12) to a hydrophobic column equilibrated with a buffer containing 1.0 M ammonium sulfate and 10 mM sodium phosphate to bring the eluate into contact with the hydrophobic column following step (12);
   (14) washing the hydrophobic column with the buffer used for equilibration in step (13) following step (13); and
   (15) eluting the antibodies from the hydrophobic column by lowering the ammonium sulfate concentration in the buffer of step (13) following step (14).
30. The method according to 28 or 29, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
31. The method according to 30, wherein the step of virus inactivation is carried out following step (3) of 28 by adjusting the pH of the eluate obtained in step (3) to 4 or lower and allowing the eluate to stand for 30 minutes or longer.
32. The method according to 30 or 31, wherein the step of virus removal via filtration with the use of a virus removal filter and/or the step of aseptic filtration are carried out following step (11) of 28.
33. The method according to 30 or 31, wherein the step of virus removal via filtration with the use of a virus removal filter and/or the step of aseptic filtration are carried out following step (15) of 29.
34. The method according to any one of 28 to 33, wherein a 1.0 M Tris-HCl buffer is used instead of the sodium phosphate buffer of step (4) of 28.
35. The method according to any one of 28 to 33, wherein the following step is carried out instead of step (4) and step (5) of 28:
   a step of adjusting the pH of an eluate to 8.0 with a 1.0 M Tris-HCl buffer following step (3).
36. A method for separating and purifying antibodies from an antibody-containing mixture comprising at least the following steps of:
   (1) applying an antibody-containing mixture to a protein A column equilibrated with a buffer of pH 6.0 to 8.5 to bring the mixture into contact with the protein A column;
   (2) washing the protein A column with the buffer of pH 6.0 to 8.5 following step (1);
   (3) eluting the antibodies with a buffer of pH 2.7 to 4.0 following step (2);
   (4) adjusting the pH of an eluate to 4.0 to 8.5 following step (3);
   (5) adjusting the pH of the eluate of step (4) to 6.0 to 8.5 following step (4);
   (6) applying the eluate of step (5) to an anion exchange column equilibrated with a buffer of pH 6.0 to 8.5 to bring the eluate into contact with the anion exchange column following step (5);
   (7) eluting the antibodies from the anion exchange column with the buffer of pH 6.0 to 8.5 following step (6);
   (8) adjusting the pH of the eluate of step (7) to 4.0 to 7.0 following step (7);
   (9) applying the eluate of step (8) to a cation exchange column equilibrated with a buffer of pH 4.0 to 7.0 to bring the eluate into contact with the cation exchange column following step (8);
   (10) washing the cation exchange column with a buffer of pH 4.0 to 7.0 following step (9); and
   (11) eluting the antibodies from the cation exchange column with a buffer of pH 4.0 to 7.0 with a salt concentration of 0.1 M to 1.0 M following step (10).
37. A method for separating and purifying antibodies from an antibody-containing mixture, which further comprises the steps of:
   (12) applying salt to the eluate of step (11) of 36 to bring the salt concentration thereof to 0.8 M to 1.2 M and adjusting the pH thereof to 6.0 to 8.0;
   (13) applying the eluate of step (12) to a hydrophobic column equilibrated with a buffer of pH 6.0 to 8.0 containing the salt of step (12) at 0.8 M to 1.2 M to bring the eluate into contact with the hydrophobic column;
   (14) washing the hydrophobic column with the buffer of pH 6.0 to 8.0 containing the salt of step (12) at 0.8 M to 1.2 M; and
   (15) eluting the antibodies from the hydrophobic column by lowering the salt concentration in the buffer of step (13).
38. The method according to 36 or 37, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
39. The method according to 38, wherein the step of virus inactivation is carried out following step (3) of 36 by adjusting the pH of the eluate obtained in step (3) to 4 or lower and allowing the eluate to stand for 30 minutes or longer.
40. The method according to 38 or 39, wherein the step of virus removal via filtration with the use of a virus removal filter and/or the step of aseptic filtration are carried out following step (11) of 36.
41. The method according to 38 or 39, wherein the step of virus removal via filtration with the use of a virus removal filter and/or the step of aseptic filtration are carried out following step (15) of 37.
42. The method according to 36, wherein the buffer of pH 6.0 to 8.5 used in step (2) is identical to the buffer used in step (1), the buffer of pH 6.0 to 8.5 used in step (7) is identical to the buffer used in step (6), and the buffer of pH 4.0 to 7.0 used in step (10) is identical to the buffer used in step (9).
43. The method according to any one of 37 to 42, wherein the buffer of pH 6.0 to 8.0 containing the salt of step (12) used in step (14) of 37 at 0.8 M to 1.2 M is identical to the buffer used in step (13).
44. The method according to any one of 36 to 43, wherein the salt used in step (11) of 36 is sodium chloride.
45. The method according to any one of 36 to 44, wherein the salt used in step (12) of 37 is ammonium sulfate.
46. The method according to any one of 36 to 45, wherein the salt concentration of the buffer used in step (1) of 36 is 4.0 M or lower.
47. The method according to 46, wherein the salt is sodium chloride.
48. A method for separating and purifying antibodies from an antibody-containing mixture comprising at least the following steps of:
   (1) applying an antibody-containing mixture to an anion exchange column equilibrated with a buffer of pH 6.0 to 8.5 to bring the mixture into contact with the anion exchange column;
   (2) eluting the antibodies from the anion exchange column with a buffer of pH 6.0 to 8.5 following step (1);
   (3) adjusting the pH of the eluate of step (2) to 4.0 to 7.0 following step (2);
   (4) applying the eluate of step (3) to a cation exchange column equilibrated with a buffer of pH 4.0 to 7.0 to bring the eluate into contact with the cation exchange column following step (3);
   (5) washing the cation exchange column with a buffer of pH 4.0 to 7.0 following step (4); and
   (6) eluting the antibodies from the cation exchange column with a buffer of pH 4.0 to 7.0 with a salt concentration of 0.1 M to 1.0 M following step (5).
49. The method according to 48, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
50. The method according to 49, wherein the step of virus inactivation is carried out before step (1) of 48 by adjusting the pH of the antibody-containing solution to 4 or lower and allowing the eluate to stand for 30 minutes or longer.
51. The method according to 49 or 50, wherein the step of virus removal via filtration with the use of a virus removal filter and/or the step of aseptic filtration are carried out following step (6) of 48.
52. The method according to any one of 48 to 51, wherein the buffer of pH 6.0 to 8.5 used in step (2) of 48 is identical to the buffer used in step (1) and the buffer of pH 4.0 to 7.0 used in step (5) of 48 is identical to the buffer used in step (4).
53. The method according to any one of 48 to 52, wherein the salt used in step (6) of 48 is sodium chloride.
54. The method according to any one of 1 to 53, wherein the antibodies are human antibodies.
55. The method according to any one of 1 to 54, wherein the antibodies are monoclonal antibodies.
56. The method according to any one of 1 to 55, wherein the antibodies are IgG antibodies.
57. The method according to 56, wherein the antibodies are IgG1, IgG2, or IgG4 antibodies.
58. The method according to any one of 54 to 57, wherein the antibodies have an amino acid sequence derived from the amino acid sequence of the heavy chain constant region by deletion, substitution, or addition of at least one amino acid residue in the naturally occurring heavy chain constant region.
59. The method according to any one of 54 to 58, wherein the antibodies are covalently or coordinately bound to other compounds.
60. A method for separating human antibodies from ungulate antibodies via protein A affinity chromatography from a mixture containing human antibodies and ungulate antibodies.
61. The method according to 60, wherein the ungulate is selected from the group consisting of a bovine, a goat, and a sheep.
62. The method according to 60 or 61, wherein separation is carried out via pH gradient elution.
63. The method according to 60 or 61, wherein separation is carried out via pH stepwise elution.
64. A method for isolating human antibodies from a mixture containing human antibodies and ungulate antibodies comprising at least the following steps of:
   (1) applying a mixture containing human antibodies and ungulate antibodies to a protein A column equilibrated with a buffer containing disodium phosphate, sodium acetate, glycine, and sodium chloride to bring the mixture into contact with the protein A column;
   (2) washing the protein A column with the buffer of step (1) following step (1); and
   (3) eluting the human antibodies from the protein A column by lowering the pH of the buffer of step (1) following step (2).
65. The method according to 64, wherein the ungulate is selected from the group consisting of a bovine, a goat, and a sheep.
66. The method according to 64 or 65, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
67. The method according to 66, wherein the step of virus inactivation is carried out following step (3) of 64 by adjusting the pH of the eluate obtained in step (3) to 4 or lower and allowing the eluate to stand for 30 minutes or longer.
68. The method according to 66 or 67, wherein the step of virus removal via filtration with the use of a virus removal filter and/or the step of aseptic filtration are carried out so as to comprise the final step of the method for isolating human antibodies from a mixture containing human antibodies and ungulate antibodies.
69. The method according to any one of 64 to 68, wherein the pH level is lowered in a gradient manner.
70. The method according to any one of 64 to 68, wherein the pH level is lowered stepwise.
71. A method for isolating human antibodies from a mixture containing human antibodies and ungulate antibodies comprising at least the following steps of:
   (1) applying a mixture containing human antibodies and ungulate antibodies to a protein A column equilibrated with a buffer containing 0.05 M to 0.15 M disodium phosphate, 0.05 M to 0.15 M sodium acetate, 0.05 M to 0.15 M glycine, and 0.05 M to 0.20 M sodium chloride to bring the mixture into contact with the protein A column;
   (2) washing the protein A column with the buffer of step (1) following step (1); and
   (3) eluting the human antibodies from the protein A column by lowering the pH of the buffer of step (1) following step (2).
72. The method according to 71, wherein the ungulate is selected from the group consisting of a bovine, a goat, and a sheep.
73. The method according to 71 or 72, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
74. The method according to 73, wherein the step of virus inactivation is carried out following step (3) of 71 by adjusting the pH of the eluate obtained in step (3) to 4 or lower and allowing the eluate to stand for 30 minutes or longer.
75. The method according to 73 or 74, wherein the step of virus removal via filtration with the use of a virus removal filter and/or the step of aseptic filtration are carried out so as to comprise the final step of the method for isolating human antibodies from a mixture containing human antibodies and ungulate antibodies.
76. The method according to any one of 71 to 75, wherein the pH level is lowered in a gradient manner.
77. The method according to any one of 71 to 75, wherein the pH level is lowered stepwise.
78. A method for isolating human antibodies from a mixture containing human antibodies and ungulate antibodies comprising at least the following steps of:
   (1) applying a mixture containing human antibodies and ungulate antibodies to a protein A column equilibrated with a buffer containing 0.10 M disodium phosphate, 0.10 sodium acetate, 0.10 M glycine, and 0.15 M sodium chloride to bring the mixture into contact with the protein A column;
   (2) washing the protein A column with the buffer of step (1) following step (1); and
   (3) eluting the human antibodies from the protein A column by lowering the pH of the buffer of step (1) following step (2).
79. The method according to 78, wherein the ungulate is selected from the group consisting of a bovine, a goat, and a sheep.
80. The method according to 78 or 79, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
81. The method according to 80, wherein the step of virus inactivation is carried out following step (3) of 78 by adjusting the pH of the eluate obtained in step (3) to 4 or lower and allowing the eluate to stand for 30 minutes or longer.
82. The method according to 80 or 81, wherein the step of virus removal via filtration with the use of a virus removal filter and/or the step of aseptic filtration are carried out so as to comprise the final step of the method for isolating human antibodies from a mixture containing human antibodies and ungulate antibodies.
83. The method according to any one of 78 to 82, wherein the pH level is lowered in a gradient manner.
84. The method according to any one of 78 to 82, wherein the pH level is lowered stepwise.
85. A method for isolating human antibodies from a mixture containing human antibodies and ungulate antibodies comprising at least the following steps of:
   (1) applying a mixture containing human antibodies and ungulate antibodies to a protein A column equilibrated with a buffer of pH 7.5 to 8.5 to bring the mixture into contact with the protein A column;
   (2) washing the protein A column with a buffer of pH 7.5 to 8.5; and
   (3) eluting the human antibodies from the protein A column by lowering the pH.
86. The method according to 85, wherein the ungulate is selected from the group consisting of a bovine, a goat, and a sheep.
87. The method according to 85 or 86, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.
88. The method according to 87, wherein the step of virus inactivation is carried out following step (3) of 85 by adjusting the pH of the eluate obtained in step (3) to 4 or lower and allowing the eluate to stand for 30 minutes or longer.
89. The method according to 87 or 88, wherein the step of virus removal via filtration with the use of a virus removal filter and/or the step of aseptic filtration are carried out so as to comprise the final step of the method for isolating human antibodies from a mixture containing human antibodies and ungulate antibodies.
90. The method according to any one of 85 to 89, wherein the pH level is lowered in a gradient manner.
91. The method according to any one of 85 to 89, wherein the pH level is lowered stepwise.
92. The method according to any one of 85 to 91, wherein the buffer of pH 7.5 to 8.5 used in step (2) of 85 is identical to the buffer used in step (1).
93. The method according to any one of 85 to 92, wherein a salt concentration of the buffer of pH 7.5 to 8.5 used in step (1) of 85 is 0.05 M to 0.20 M.
94. The method according to 93, wherein the salt is sodium chloride.
95. The method according to any one of 60 to 94, wherein the human antibodies are human polyclonal antibodies.
96. The method according to any one of 60 to 95, wherein the ungulate antibodies are selected from the group consisting of bovine polyclonal antibodies, goat polyclonal antibodies, and sheep polyclonal antibodies.
97. The method according to any one of 60 to 96, wherein the human antibodies are IgG antibodies.
98. The method according to 97, wherein the human antibodies are IgG1 antibodies, IgG2 antibodies, or IgG4 antibodies.

This description includes part or all of the contents as disclosed in the description and/or drawings of Japanese Patent Application No. 2003-097407, which is a priority document of the present application.

### Brief Description of the Drawings

Fig. 1 shows the elution curve of human polyclonal antibodies from a protein A affinity chromatography column.
Fig. 2 shows the elution curve of bovine polyclonal antibodies from a protein A affinity chromatography column.
Fig. 3 shows the elution curve of a mixture of human polyclonal antibodies and bovine polyclonal antibodies from a protein A affinity chromatography column.
Fig. 4 shows the elution curve of goat polyclonal antibodies from a protein A affinity chromatography column.
Fig. 5 shows the elution curve of a mixture of human polyclonal antibodies and goat polyclonal antibodies from a protein A affinity chromatography column.
Fig. 6 shows the elution curve of sheep polyclonal antibodies from a protein A affinity chromatography column.
Fig. 7 shows the elution curve of a mixture of human polyclonal antibodies and sheep polyclonal antibodies from a protein A affinity chromatography column.

### Preferred Embodiments of the Invention

Hereafter, the present invention is described in detail.

In the method of separating and purifying the antibodies of the present invention from a mixture containing such antibodies, antibody-containing mixtures include, for example, culture supernatants of antibody-producing hybridomas, myeloma cells such as NSO, animal cells that have been transformed by an antibody-encoding gene and are capable of expressing and producing such antibodies, and yeast. When purification is conducted by the method of the present invention, such culture supernatants are preferably clarified. Clarification may be carried out by, for example, filtration through a 0.2 µm-membrane filter. The antibodies purified in the present invention are not limited. Examples thereof include human antibodies, nonhuman animal antibodies, such as mouse antibodies and ungulate antibodies derived from a bovine, a goat, or a sheep, chimeric antibodies of humans and nonhuman animals, and humanized antibodies prepared by humanizing nonhuman animal antibodies. Human antibodies are preferable, and human monoclonal antibodies are more preferable. Also, classes or subclasses of antibodies are not particularly limited, and antibodies of any classes or subclasses can be purified according to the method of the present invention. IgG antibodies are preferable, and IgG1, IgG2, and IgG4 antibodies are particularly preferable. Antibodies may have an amino acid sequence derived from the amino acid sequence of the heavy chain constant region by deletion, substitution, or addition of at least one amino acid residue in the naturally occurring heavy chain constant region. The antibodies are covalently or coordinately bound to other compounds.

In the present invention, a mixture that contains at least the aforementioned antibodies is purified via anion exchange chromatography and cation exchange chromatography in that order. Such mixture may be purified via protein A affinity chromatography prior to purification via anion exchange chromatography. Alternatively, such mixture may be purified via cation exchange chromatography followed by hydrophobic chromatography. Further, protein G affinity chromatography may be carried out instead of protein A affinity chromatography. Furthermore, gel filtration, hydroxyapatite chromatography, or metal chelate affinity chromatography may be carried out instead of hydrophobic chromatography.

Such procedures of chromatography involve the use of a column containing anion exchange resins, a column containing cation exchange resins, a column containing protein A-bound resins, and a column containing resins to which a hydrophobic ligand had been bound. A commercially available column for fractionation chromatography may be employed. Alternatively, a commercially available empty column or an empty column prepared from a glass tube may be filled with the aforementioned resins and then used. Commercially available resins may be employed.

Examples of commercially available protein A columns include MabSelect and Protein A Sepharose FF (Amersham Biosciences), Prosep rA and Prosep A (Millipore), and POROS A/20 (Applied Biosystems). Examples of commercially available anion exchange columns include Q Sepharose XL, Q Sepharose FF, DEAE Sepharose FF, and ANX Sepharose FF (Amersham Biosciences). Examples of commercially available cation exchange columns include SP Sepharose FF and CM Sepharose FF (Amersham Biosciences) and Poros 50 HS (Applied Biosystems). Examples of commercially available hydrophobic columns include Phenyl Sepharose HP, Phenyl Sepharose FF, and Butyl Sepharose FF (Amersham Biosciences) and Phenyl Toyopearl 650 S and Phenyl Toyopearl 650 M (Tosoh).

Cellulose, agarose, dextran, silica, synthetic polymers, or other types of resins, such as Sepharose, Sephadex, or Cellulofine, to which an ion exchange group has been bound may be employed as ion exchange chromatography resins for production of columns. As an anion exchange group, a diethylaminoethyl (DEAE) group or a quaternary aminoethyl (QAE) group that has stronger basicity than DEAE may be employed. As a cation exchange group, a carboxymethyl (CM) group, a sulfopropyl (SP) group, or a phosphate group that has stronger acidity than CM may be selected. Examples of hydrophobic ligands that are bound to resins for hydrophobic chromatography include phenyl, butyl, octyl, and ethyl, and any thereof can be employed.

Hereafter, methods of purification via each type of chromatography are described. In the following description, a case where 220 ml to 1,500 ml of a mixture containing antibodies of interest at a concentration of 700 mg/l is used is exemplified. The concentration and the amount of antibodies to be employed are not limited. The size of column, the amount of buffer to be used, and other conditions can be adequately modified according to the concentration and the amount of the antibodies.

When anion exchange chromatography is carried out, a column is previously equilibrated with a buffer of pH 6 to 8.5, and preferably a buffer of pH 8.0. Examples of buffers used in such a case include Tris-HCL (2-amino-2-hydroxymethyl-1,3-propanediol) and Bis-Tris propane (1,3-bis[tris(hydroxymethyl)-methylamino]propane). In this case, a buffer concentration of 10 mM to 20 mM is preferable, and a buffer concentration of 10 mM is more preferable. Whether or not the column has been equilibrated may be confirmed by determining whether the pH level and the conductivity of the buffer that had passed through the column are the same as those of the buffer of the initial state. The column size may vary depending on the volume of the antibody-containing mixture that is applied as a sample. For example, use of a column having a length of 2.5 cm to 30 cm is sufficient. The pH level of the antibody-containing mixture that is applied to an anion exchange column or antibody-containing mixture that has been purified via protein A affinity chromatography is adjusted to between 6.0 and 8.5, and preferably to 8.0. In such a case, the type of buffer used for adjusting the pH level is not limited, and sodium phosphate buffer, Tris-HCl buffer, or the like is used. Use of the same type of buffer used for equilibrating the anion exchange column is preferable. It is preferable to adjust the pH level of the buffer for equilibration to the same level as that of the antibody-containing buffer. Alternatively, the pH level of the buffer for equilibration is preferably set somewhat higher. After the sample has been applied, a buffer of pH 6.0 to 8.5, and preferably the buffer for equilibration, in amounts of several column volumes, and preferably about 3 column volumes, is allowed to flow through the column for elution. Fractions that did not adsorb to the column contain antibodies of interest. The linear flow rate of the buffer varies depending on the column size, and such rate may be selected from rates within the range of 50 cm/h to 500 cm/h.

Subsequently, fractions that did not adsorb to the anion exchange column are purified via cation exchange chromatography. When cation exchange chromatography is carried out, the column is previously equilibrated with a buffer of pH 4.0 to 7.0, and preferably with a buffer of pH 5.0. Examples of a buffer used include sodium acetate buffer, sodium citrate buffer, sodium phosphate buffer, and MES (2-morpholinoethanesulfonic acid). In such a case, a buffer concentration of 10 mM to 50 mM is preferable, and a buffer concentration of 20 mM is more preferable. Whether or not the column has been equilibrated may be confirmed by determining whether the pH level and the conductivity of the buffer that had passed through the column are the same as those of the buffer of the initial state. The column size may vary depending on the volume of the antibody-containing mixture that is applied as a sample. For example, use of a column having a length of 2.5 cm to 30 cm is sufficient. The fraction that did not adsorb to the anion exchange column, which is applied to the cation exchange chromatography column, is adjusted to a pH level of 4.0 to 7.0, and preferably to a pH level of 5.0. In this case, a solution and a buffer used for adjusting a pH level can be adequately selected. Examples thereof include acetic acid, citric acid and an acetate-citrate buffer. When adjusting pH level with the use of acetic acid, the acetic acid concentration is preferably 0.5 M to 1.5 M, and more preferably 1.0 M. The pH level of the buffer for equilibration of the cation exchange column is preferably adjusted to the same level as the pH level of the fraction that did not adsorb to the anion exchange column. Alternatively, the pH level of the buffer for equilibration is preferably set somewhat lower. After the fraction that did not adsorb to the anion exchange column has been applied, the column is washed with several column volumes, and preferably about 5 column volumes, of the buffer. An example of buffer used for washing is a buffer of pH 4.0 to 7.0, and use of such buffer for equilibration is preferable. Subsequently, several column volumes, and preferably about 5 column volumes, of a buffer with a 0.1 M to 1.0 M salt content is allowed to flow through the column to elute the antibodies of interest. In such a case, use of a buffer prepared by adding 0.1 M to 1.0 M, and preferably 0.3 M, of sodium chloride to the buffer for equilibration is preferable. For example, a buffer containing 10 mM to 50 mM, and preferably 20 mM, sodium acetate is sufficient. Alternatively, a buffer containing 20 mM sodium phosphate and 10 mM citrate may be used instead of a buffer containing 20 mM sodium acetate.

Salt can be adequately selected and, for example, potassium chloride can be used besides sodium chloride. A fraction that did not adsorb to the column contains antibodies of interest. The linear flow rate of the buffer varies depending on the column size, and such rate may be selected within the range of 50 cm/h to 500 cm/h. In this case, the antibodies of interest may be eluted by salt concentration gradient elution by increasing stepwise the sodium chloride concentration. The gradient volume is 5 to 40 column volumes and preferably 20 column volumes. In order to conduct concentration gradient elution, an automatic mixing apparatus or a gradient-preparing apparatus having two solvent tanks may be used. Further, salt concentration may be increased stepwise. The term "gradient elution" refers to a form of elution by continually varying the concentration or composition of the solvent that is allowed to flow through the column. The term "stepwise elution" refers to a form of elution by intermittently replacing the solvent that is allowed to flow through the column with a solvent having different concentration or composition and higher capacity for elution. The term "pH gradient elution" refers to a form of elution that is carried out by continually changing the pH levels of the solvent, and the term "pH stepwise elution" refers to a form of elution that is carried out by intermittently changing the pH levels of the solvent. The term "gradient manner" means that change occurs continually, and the term "stepwise" means that change occurs intermittently.

When purification via protein A affinity chromatography is carried out prior to purification via anion exchange chromatography, such procedures are carried out in the following manner. The column is previously equilibrated with a buffer of pH 6.0 to 8.5, and preferably pH 7.0. Examples of a buffer that can be used for equilibration include sodium phosphate, potassium phosphate, HEPES (2-[4-(2-hydroxyethyl)-1-piperazinyl]ethanesulfonic acid), and MES (2-morpholinoethanesulfonic acid). More preferably, such buffer contains 0 M to 4.0 M sodium chloride or potassium chloride. Examples thereof include a buffer containing 10 mM to 100 mM, and preferably 10 mM to 20 mM, sodium phosphate, and 0 M to 4.0 M, and preferably 0.15 M, sodium chloride. Whether or not the column has been equilibrated may be confirmed by determining whether the pH level and the conductivity of the buffer that had passed through the column are the same as those of the buffer of the initial state. The column size may vary depending on the amount of the antibody-containing mixture that is applied as a sample. For example, use of a column having a length of 5 cm to 30 cm is sufficient. After the antibody-containing mixture has been applied, the column is washed with a buffer of, for example, pH 6.0 to 8.5, and preferably a buffer of pH 6.0 to 7.0. In this case, use of the buffer for equilibration is preferable. The amount of the buffer to be used is not limited, and washing may be carried out with the use of, for example, 5 column volumes of the buffer. The linear flow rate of the buffer varies depending on the column size, and such rate may be selected within the range of 50 cm/h to 1,000 cm/h. Subsequently, antibodies are eluted from the column with the aid of a buffer of pH 2.7 to 4.0, and preferably pH 3.4 to 3.5, such as a sodium citrate buffer. The concentration of the sodium citrate buffer is preferably 10 mM to 100 mM, and more preferably 20 mM. The amount of the buffer to be used is not limited, and washing may be carried out with the use of, for example, 5 column volumes of the buffer. The linear flow rate of the buffer varies depending on the column size, and such rate may be selected within the range of 50 cm/h to 1,000 cm/h. The eluate contains antibodies of interest. The eluate obtained via protein A affinity chromatography is adjusted to pH 4.0 to 8.5, and preferably pH 8.0, in order to carry out the subsequent purification via anion exchange chromatography. Examples of the buffer include a sodium phosphate buffer and a Tris-HCl buffer.

In this case, both a sodium phosphate buffer and a Tris-HCl buffer may be used. For example, the sodium phosphate buffer may first be added, and the Tris-HCl buffer may then be added. Alternatively, either the sodium phosphate buffer or the Tris-HCl buffer may be used. The concentration of the sodium phosphate buffer and that of the Tris-HCl buffer are 10 mM to 200 mM, preferably 0.5 M to 1.5 M, and more preferably 100 mM or 1.0 M, respectively.

Purification via protein A affinity chromatography, anion exchange chromatography, and cation exchange chromatography results in antibody purity of 98%, and the yield from purification is approximately 70%. Antibody purity can be measured via, for example, gel filtration HPLC.

Further, purification may be carried out in the following manner via the procedure composed of the aforementioned cation exchange chromatography followed by hydrophobic chromatography.

The column is previously equilibrated with a buffer of pH 6.0 to 8.0 with a salt content of 0.8 M to 1.2 M. A buffer used for equilibration has, for example, a pH of 6.0 to 8.0, and preferably a pH of 7.0, and such buffer contains 0.8 M to 1.2 M, and preferably 1.0 M, ammonium sulfate. Examples thereof include buffers containing 10 mM to 100 mM, and preferably 10 mM, sodium phosphate. Salt is added to the cation exchange chromatography eluate to a salt content of 0.8 M to 1.2 M, pH level is adjusted to 6.0 to 8.0, and the resultant is then applied to the column. Such adjustment is carried out by, for example, adding ammonium sulfate to 1.0 M, and adding an aqueous solution of sodium hydroxide in order to adjust the pH level to 6.0 to 8.0, and preferably to 7.0, according to need. Ammonium sulfate is added to an ammonium sulfate concentration of 0.8 M to 1.2 M, and preferably 1.0 M, and the concentration of the aqueous solution of sodium hydroxide is 1.0 M to 10.0 M, and preferably 5.0 M. The column size may vary depending on the amount of the antibody-containing mixture that is applied as a sample. For example, use of a column having a length of 2.5 cm to 20 cm is sufficient. After the antibody-containing mixture is applied, the column is washed with a buffer for equilibration, such as a buffer with a pH of 6.0 to 8.0 with a salt content of 0.8 M to 1.2 M to rinse off the fractions that did not adsorb to the column. The amount of the buffer is not limited. For example, the column may be washed with 5 column volumes of buffer. The linear flow rate of the buffer varies depending on the column size, and such rate may be selected within the range of 50 cm/h to 200 cm/h. Subsequently, salt concentration gradient elution is carried out wherein the concentration of ammonium sulfate in 10 mM to 50 mM, and preferably 10 mM, the sodium phosphate buffer is lowered stepwise. In this case, the gradient volume is 5 to 30 column volumes, and preferably 10 column volumes. According to need, the salt concentration may be raised constantly or stepwise. The linear concentration may be selected within the range of 50 cm/h to 200 cm/h.

Via hydrophobic chromatography, antibody purity becomes 99% or higher.

In accordance with the type of antibodies to be purified, a step of washing may be additionally carried out prior to a step of chromatography. In the case of protein A affinity chromatography, for example, a step of washing with a buffer (pH 6.0) containing 20 mM sodium phosphate and 1 M sodium chloride and/or a buffer (pH 6.0) containing 10 mM sodium phosphate may be carried out before eluting antibodies. Thus, nonspecific substances adsorbed to the column can be removed. The amount of the buffer used is not limited. For example, the column may be washed with 5 column volumes of buffer. In the case of cation exchange chromatography, for example, the column is washed with a 10 mM sodium phosphate buffer (pH 6.0, 6.5, or 7.0) before eluting antibodies to remove impurities. The amount of the buffer used is not limited. For example, the column may be washed with 5 column volumes of the buffer.

The antibody-containing culture supernatant occasionally contains viruses derived from antibody-producing cells. Accordingly, the method of purifying antibodies according to the present invention may include a step of virus inactivation and/or virus removal. For the purpose of virus inactivation, a solution that contains the antibodies obtained through the process of purification may be allowed to stand under acidic conditions for a given period of time. For example, an acidic solution such as a citric acid solution may be added thereto and the resultant may be allowed to stand for a given period of time. A step of virus inactivation may be carried out at any stage of the purification procedure. Since a citrate buffer is used when eluting antibodies from the protein A column, a concentrated citric acid solution, such as a 1 M citric acid solution, is added to the aforementioned eluate to adjust the pH level to 4 or lower, and preferably 3.5 or lower. The resultant may then be allowed to stand for at least 15 minutes, preferably at least 30 minutes, and more preferably at least 45 minutes. When the step of virus inactivation is carried out under acidic conditions, the concentration and the pH of the buffer used for the step immediately before the step of virus inactivation may be preferably lowered so that acidic conditions can be easily attained. When virus inactivation is carried out following the step of purification via protein A column, for example, the protein A column is generally equilibrated with a 20 mM phosphate buffer of pH 7. In contrast, a 10 mM phosphate buffer of pH 6 may be used. While elution is generally carried out with the use of a citrate buffer of pH 3.5, a citrate buffer of pH 3.4 may be used.

In the method of the present invention, the antibody-containing solution eluted from the protein A column is neutralized and is then subjected to anion exchange chromatography. When virus inactivation is carried out using a citric acid solution, however, the pH level or the concentration of the buffer for neutralization must be increased. When the step of virus inactivation is not carried out, the antibody-containing solution eluted from the protein A column is adjusted to a pH of 7.0 with a 100 mM sodium phosphate buffer, and is then adjusted to a pH of 8.0 with a 1.0 M Tris-HCl buffer. In such a case, a 10 mM sodium phosphate buffer of pH 8.2 may be added, and a 1.5 M Tris-HCl buffer may then be added to adjust the pH level to 8.0.

Virus removal can be carried out via filtration using a virus removal filter. An example of a virus removal filter is Planova® 20N (Asahi Kasei Corporation). The purified antibodies obtained by the method of the present invention are subjected to concentration or aseptic filtration according to need. The step of concentration may be carried out using, for example, an ultrafilter membrane. Further, a buffer may be replaced with an adequate buffer during the step of concentration for stabilizing antibodies. For example, a buffer may be replaced with a 1 mM to 50 mM glutamate buffer. Also, sorbitol or the like may be incorporated in the replaced buffer in order to stabilize the antibodies. The step of aseptic filtration may be carried out using, for example, a membrane filter having a pore diameter of 0.45 µm or smaller. The step of virus removal, the step of concentration, and the step of aseptic filtration may be carried out at any stage in the process of purification according to the present invention. These processes are preferably carried out using the purified antibodies after the completion of purification thereof; that is, these processes are preferably carried out at the end of the purification process. For example, the step of virus removal and the step of aseptic filtration may be carried out after antibodies are eluted via cation exchange chromatography. When the process of purification comprises a step of purification via hydrophobic chromatography, the step of virus removal and the step of aseptic filtration may be carried out after antibodies are eluted via hydrophobic chromatography. The order of carrying out the step of virus removal and the step of aseptic filtration is not limited; however, it is preferable to carry out the step of aseptic filtration following the step of virus removal through a virus removal filter.

The present invention includes a method for separating human antibodies from ungulate antibodies, such as bovine, sheep, or goat antibodies, from a mixture containing human antibodies and such ungulate antibodies. Human and bovine antibodies may be monoclonal or polyclonal antibodies, and polyclonal antibodies are preferable. For example, the present invention can be utilized to separate human monoclonal antibodies cultured in bovine serum-containing medium, chimeric antibodies of human and nonhuman animals, and humanized antibodies prepared by humanizing nonhuman animal antibodies from bovine antibodies in the bovine serum from CHO cells, NSO myeloma cells, or the culture supernatant of hybridomas that are capable of producing these antibodies. The present invention can also be utilized to separate human monoclonal antibodies, chimeric antibodies of human and nonhuman animals, and humanized antibodies prepared by humanizing nonhuman animal antibodies from the body fluids of the transgenic bovine capable of producing such antibodies, such as blood or emulsions. Further, the present invention can be utilized to separate human polyclonal antibodies from bovine-derived antibodies. When a transgenic bovine capable of producing human antibodies to which the locus of the human antibodies has been introduced is immunized with an antigen, for example, bovine body fluids such as blood and emulsions contain human polyclonal antibodies and bovine polyclonal antibodies. The present invention can be utilized to separate these antibodies from such mixture.

Human antibodies are separated from ungulate antibodies such as bovine, sheep, or goat antibodies via protein A affinity chromatography. The column size and the volume of antibody mixture used can be adequately determined. The protein A column is equilibrated with a buffer of pH 7.5 to 8.5, and preferably a buffer of pH 8.0. Examples of buffers that can be used include a buffer containing disodium phosphate and a buffer containing sodium acetate, glycine, and sodium chloride. The concentration of disodium phosphate, sodium acetate, and glycine in the buffer is preferably 0.05 M to 0.15 M, and is more preferably 0.10 M. The concentration of sodium chloride is preferably 0.05 M to 0.20 M, and more preferably 0.15 M. A specific example of such buffer is a buffer (pH 8.0) containing 0.10 M disodium phosphate, 0.10 M sodium acetate, 0.10 M glycine, and 0.15 M sodium chloride. Whether or not the column has been equilibrated may be confirmed by determining whether the pH level and the conductivity of the buffer that had passed through the column are the same as those of the buffer of the initial state. An antibody mixture containing human antibodies and, for example, bovine antibodies, the pH level of which has been adjusted preferably with the use of the same buffer used for equilibration, is applied to the equilibrated column. In this case, the volume or concentration of the buffer to be added for pH adjustment may be adequately determined in accordance with the antibody concentration in the antibody mixture or other conditions. Subsequently, the column is washed with a buffer used for equilibration, such as a buffer of pH 7.5 to 8.5, and preferably a buffer of pH 8.0. The amount of buffer used is not limited. For example, the column may be washed with 5 column volumes of buffer. Thereafter, gradient elution is carried out wherein a buffer (pH 2.5) containing 0.10 M sodium acetate, 0.10 M glycine, and 0.15 M sodium chloride is gradually added to a buffer (pH 8.0), which contains 0.10 M disodium phosphate, 0.10 M sodium acetate, 0.10 M glycine, and 0.15 M sodium chloride to gradually lower the pH level (i.e., pH gradient elution). Thus, human antibodies can be separated from bovine antibodies. The gradient volume is 20 to 40 column volumes, and preferably 30 column volumes. The lower limit of the pH level in pH gradient elution is, for example, 2.0 to 3.0, and preferably 2.5. The linear flow rate varies depending on the column size, and it may be selected within the range of 50 cm/h to 150 cm/h. In such a case, pH stepwise elution may be carried out.

The aforementioned procedures of chromatography can be automatically carried out using an automatic apparatus. For example, fast pressure liquid chromatography (FPLC) can be carried out under adequate program control.

Hereafter, the examples of the present invention are described, although the technical scope of the present invention is not limited to these examples.

### Example 1: Purification of human monoclonal antibodies

A serum free culture supernatant of CHO cells (220 ml; level of human monoclonal antibodies expressed: 700 mg/l) containing human monoclonal antibodies (human IgG1 antibodies) clarified via a depth filter and a 0.2-µm membrane filter was applied to the protein A column (MabSelect; 8 mm ID x 20 cm; Amersham Biosciences) that had been equilibrated with a buffer (pH 7.0) containing 20 mM sodium phosphate and 0.15 M sodium chloride (linear flow rate: 500 cm/h). After the culture supernatant had been applied, the column was washed with 5 column volumes of buffer for equilibration (linear flow rate: 500 cm/h). Subsequently, human monoclonal antibodies were eluted using 5 column volumes of 20 mM sodium citrate buffer (pH 3.5) (linear flow rate: 500 cm/h). A 100 mM sodium phosphate buffer (pH 8.0) was added to the eluate to bring the pH level to 7.0. This procedure was also carried out by using a 1.0 M Tris-HCl buffer instead of the 100 mM sodium phosphate buffer.

The pH of the eluate from the MabSelect was adjusted to 8.0 with the aid of 1 M Tris-HCl (pH 9.0), and the resulting eluate was applied to the anion exchange column (Q Sepharose XL; 7 mm ID x 15 cm; Amersham Biosciences) that had been equilibrated with a 10 mM Tris-HCl buffer (pH 8.0) (linear flow rate: 300 cm/h). After the completion of application, 3 column volumes of buffer for equilibration were allowed to pass through the column (linear flow rate: 300 cm/h). Fractions that did not adsorb to the column were pooled as the eluate of human monoclonal antibodies.

The pH level of the solution pooled via Q sepharose XL was adjusted to 5.0 with 1 M acetic acid, and the resultant was applied to the cation exchange column (SP Sepharose FF; 10 mm ID x 10 cm; Amersham Biosciences) that had been equilibrated with a 20 mM sodium acetate buffer (pH 5.0) (linear flow rate: 300 cm/h). After the completion of the application, the column was washed with 5 column volumes of buffer for equilibration (linear flow rate: 300 cm/h). Subsequently, 5 column volumes of buffer (pH 5.0) containing 20 mM sodium acetate and 0.3 M sodium chloride were allowed to pass through the column to elute human monoclonal antibodies (linear flow rate: 300 cm/h).

According to the method of the present invention, the yield from purification of human monoclonal antibodies was approximately 70%, and the purity of such antibodies was 98% as a result of gel filtration HPLC analysis.

In accordance with the type of antibodies, a step of washing was carried out before eluting antibodies from the column. In the case of protein A affinity chromatography, for example, a step of column washing with 5 column volumes of buffer (pH 6.0) containing 20 mM sodium phosphate and 1 M sodium chloride and/or a buffer (pH 6.0) containing 10 mM sodium phosphate was carried out before eluting human monoclonal antibodies with a sodium citrate buffer to remove nonspecific substances adsorbed to the column. In the case of cation exchange chromatography, the column was washed with 5 column volumes of 10 mM sodium phosphate buffer (pH 7.0) before eluting human monoclonal antibodies with a buffer containing 20 mM sodium acetate and 0.3 M sodium chloride to remove impurities.

In accordance with the type of antibody, a step of hydrophobic chromatography is sometimes carried out after the purification sequence of protein A affinity chromatography, anion exchange chromatography, and cation exchange chromatography.

Examples of chromatography conditions are described below.

Ammonium sulfate was added to the solution pooled via SP sepharose FF to a concentration of 1 M, and the pH level of the resulting solution was then adjusted to 7.0 with the aid of an aqueous solution of 10 M sodium hydroxide. This solution was applied to a hydrophobic column (Phenyl Sepharose HP; 10 mm ID x 10 cm; Amersham Biosciences) that had been equilibrated with a buffer (pH 7.0) containing 1 M ammonium sulfate and 10 mM sodium phosphate (linear flow rate: 160 cm/h). After the completion of the application, the column was washed with 5 column volumes of buffer for equilibration (linear flow rate: 160 cm/h). Subsequently, the ammonium sulfate content in the buffer containing 1 M ammonium sulfate and 10 mM sodium phosphate was lowered (gradient volume: 10 column volumes of buffer were used to bring the ammonium sulfate content from 1 M to 0 M) to elute human monoclonal antibodies (linear flow rate: 160 cm/h).

The purity of human monoclonal antibodies was improved to 99% or higher by carrying out the step of hydrophobic chromatography (gel filtration HPLC analysis).

### Example 2: Separation and purification of human polyclonal antibodies from bovine polyclonal antibodies

Human polyclonal antibodies (3 mg; trade name: Human IgG from Serum; Cat No. 14506; Sigma) and bovine polyclonal antibodies (3 mg; trade name: Bovine IgG from Serum; Cat No. 15506; Sigma) were each separately dissolved in 3 ml of buffer (pH 8.0) containing 0.10 M disodium phosphate, 0.10 M sodium acetate, 0.10 M glycine, and 0.15 M sodium chloride. Thereafter, the resulting solutions were allowed to pass through a 0.2-µm filter and then separately designated as the solution of human polyclonal antibodies and the solution of bovine polyclonal antibodies.

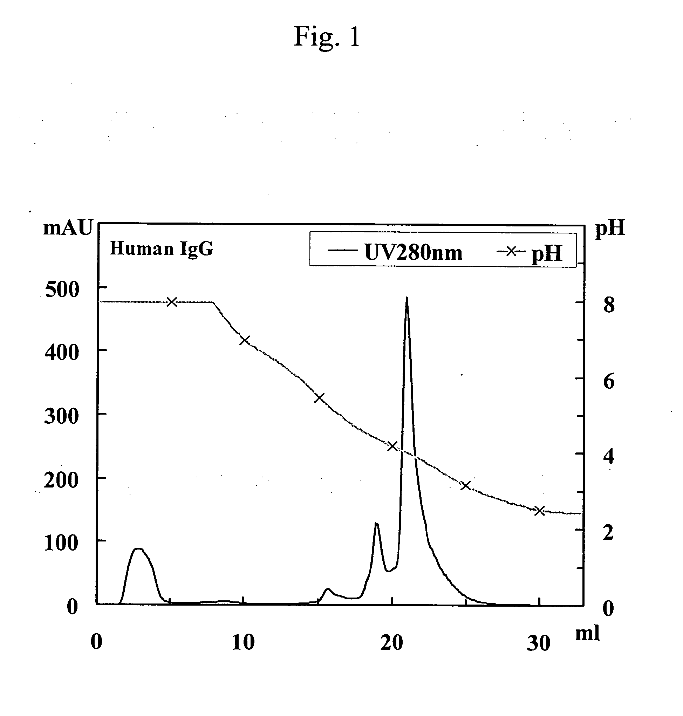

The following 3 samples were separately applied to 3 protein A columns (POROS A/20; 4.6 mm ID x 5 cm; Applied Biosystems) that had been equilibrated with a buffer (pH 8.0) containing 0.10 M disodium phosphate, 0.10 M sodium acetate, 0.10 M glycine, and 0.15 M sodium chloride (linear flow rate: 90 cm/h). The 3 samples were as follows: (i) 1 ml of solution containing human polyclonal antibodies; (ii) 1 ml of solution containing bovine polyclonal antibodies; and (iii) 2 ml of mixed solution containing equivalent amounts of human polyclonal antibodies and bovine polyclonal antibodies. These sample solutions were separately applied to the columns. After the completion of application, the columns were washed with 5 column volumes of buffer for equilibration (linear flow rate: 90 cm/h). Subsequently, human polyclonal antibodies and bovine polyclonal antibodies were eluted via pH gradient elution (gradient volume: 30 column volumes of buffer were used to bring the pH level from 8.0 to 2.5) in which the pH level was lowered stepwise by gradually adding a buffer (pH 2.5) containing 0.10 M sodium acetate, 0.10 M glycine, and 0.15 M sodium chloride to a buffer containing 0.10 M disodium phosphate, 0.10 M sodium acetate, 0.10 M glycine, and 0.15 M sodium chloride (linear flow rate: 90 cm/h). The elution curves obtained via the aforementioned chromatography techniques are shown in Figs. 1, 2, and 3.

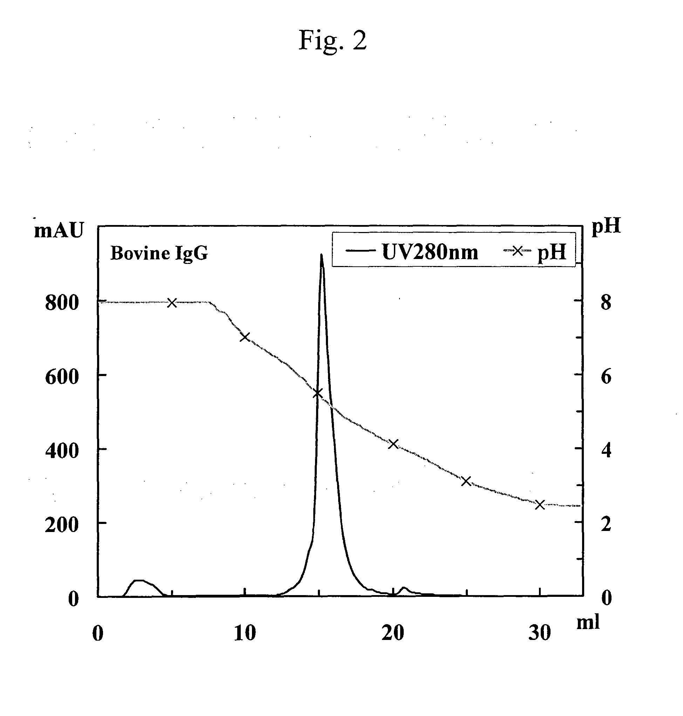

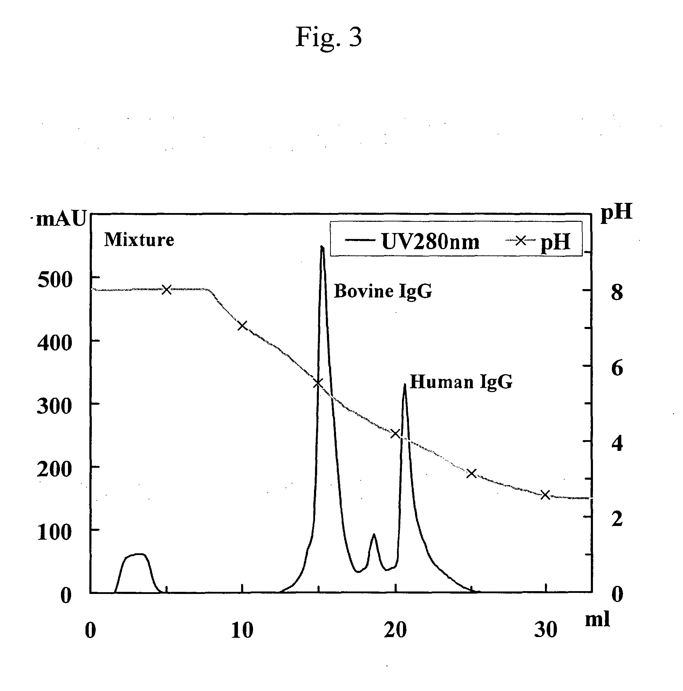

As shown in Figs. 1 and 2, the rate of elution of bovine polyclonal antibodies was faster than that of human polyclonal antibodies in the case of pH gradient elution. As shown in Fig. 3, human polyclonal antibodies were separated from bovine polyclonal antibodies via chromatography of a mixture containing human and bovine polyclonal antibodies.

### Example 3: Purification of human monoclonal antibodies II

A serum free culture supernatant of CHO cells (1,500 ml; level of human monoclonal antibodies expressed: 700 mg/l) containing human monoclonal antibodies (human IgG1 antibodies) clarified via a depth filter and a 0.2 µm-membrane filter was applied to the protein A column (MabSelect; 20 mm ID x 20 cm; Amersham Biosciences) that had been equilibrated with a buffer (pH 6.0) containing 10 mM sodium phosphate (linear flow rate: 500 cm/h). After the culture supernatant had been applied, the column was washed with 5 column volumes of buffer for equilibration (linear flow rate: 500 cm/h). Subsequently, human monoclonal antibodies were eluted using 5 column volumes of a buffer (pH 3.4) containing 20 mM sodium citrate (linear flow rate: 500 cm/h). Citric acid, 1 M, was added to the eluate to bring the pH level to 3.5 for the purpose of virus inactivation. The resulting solution was allowed to stand for 45 minutes at room temperature, and the equivalent amount of a buffer (pH 8.2) containing 10 mM sodium phosphate was added thereto for neutralization.

The pH level of the solution that had been subjected to virus inactivation was adjusted to 8.0 with the aid of 1.5 M Tris-HCl, and the resulting solution was applied to the anion exchange column (Q Sepharose XL; 10 mm ID x 15 cm; Amersham Biosciences) that had been equilibrated with a 10 mM Tris-HCl buffer (pH 8.0) (linear flow rate: 300 cm/h). After the completion of application, 3 column volumes of buffer for equilibration were allowed to pass through the column (linear flow rate: 300 cm/h). Fractions that did not adsorb to the column were pooled as the eluate of human monoclonal antibodies.

The pH level of the solution pooled via Q Sepharose XL was adjusted to 5.0 with 1 M citric acid, and the resultant was applied to the cation exchange column (SP Sepharose FF; 26 mm ID x 15 cm; Amersham Biosciences) that had been equilibrated with a buffer (pH 5.0) containing 20 mM sodium phosphate, 10 mM citric acid, and 0.05 M sodium chloride (linear flow rate: 300 cm/h). After the completion of the application, the column was washed with 5 column volumes of buffer for equilibration (linear flow rate: 300 cm/h). Subsequently, 5 column volumes of buffer (pH 5.0) containing 20 mM sodium phosphate, 10 mM citric acid, and 0.3 M sodium chloride were allowed to pass through the column to elute human monoclonal antibodies (linear flow rate: 300 cm/h).

The solution pooled by SP Sepharose FF was concentrated to 10 mg/ml via an ultrafilter membrane (Biomax 30; 50 cm²; Millipore) that had been equilibrated with a buffer (pH 5.5) containing 10 mM sodium glutamate and 262 mM D-sorbitol. Thereafter, the buffer was exchanged with the same type of buffer in an amount at least ten times the initial amount thereof. After the buffer exchange, the solution of human monoclonal antibodies was concentrated to 20 mg/ml, and the solution of antibodies was recovered.

The recovered solution of antibodies that had been subjected to concentration and buffer exchange was aseptically filtered through a 0.2-µm membrane filter (Durapore hydrophilic multimedia filter; Millipore). The resultant was designated as the final purified product.

According to the method of the present invention, the yield from purification of human monoclonal antibodies was approximately 70%, and the purity of such antibodies was 98% as a result of gel filtration HPLC analysis.

The solution pooled via SP Sepharose FF was filtered through a virus removal filter (Planova® 20N; Asahi Kasei Corporation) according to need. The filtrate was subjected to an ultrafilter membrane for concentration and buffer exchange.

In accordance with the type of antibodies, a step of washing was carried out before eluting antibodies from the column. In the case of protein A affinity chromatography, for example, a step of column washing with 5 column volumes of buffer (pH 6.0) containing 10 mM sodium phosphate and 1 M sodium chloride and/or a buffer (pH 6.0) containing 10 mM sodium phosphate was carried out before eluting human monoclonal antibodies with a sodium citrate buffer to remove nonspecific substances adsorbed to the column. In the case of cation exchange chromatography, the column was washed with 5 column volumes of 10 mM sodium phosphate buffer (pH 6.0, 6.5, or 7.0) before eluting human monoclonal antibodies with a buffer containing 20 mM sodium phosphate, 10 mM citric acid, and 0.3 M sodium chloride to remove impurities.

### Example 4: Separation and purification of human polyclonal antibodies from goat polyclonal antibodies

Human polyclonal antibodies (3 mg; trade name: Human IgG from Serum; Cat No. 14506; Sigma) and goat polyclonal antibodies (3 mg; trade name: Goat IgG from Serum; Cat No. I5256; Sigma) were each separately dissolved in 3 ml of buffer (pH 8.0) containing 0.10 M disodium phosphate, 0.10 M sodium acetate, 0.10 M glycine, and 0.15 M sodium chloride. Thereafter, the resulting solutions were allowed to pass through a 0.2-µm filter and then separately designated as the solution of human polyclonal antibodies and the solution of goat polyclonal antibodies.

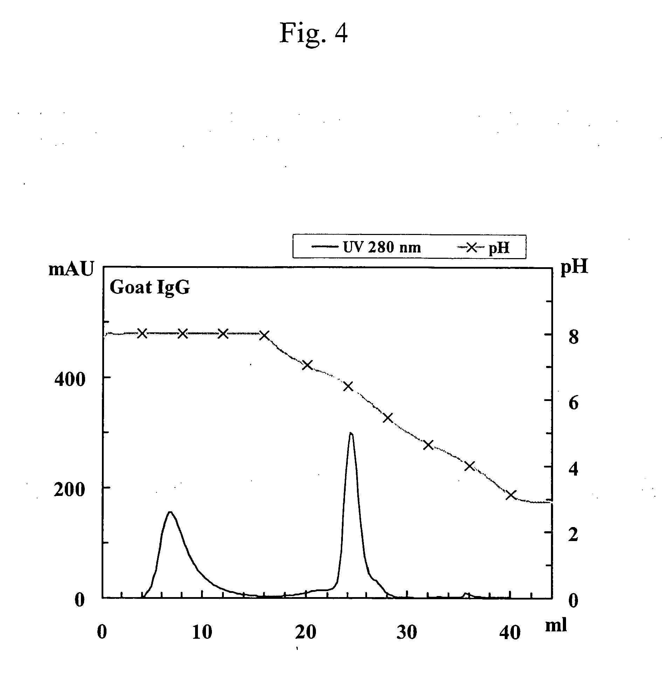

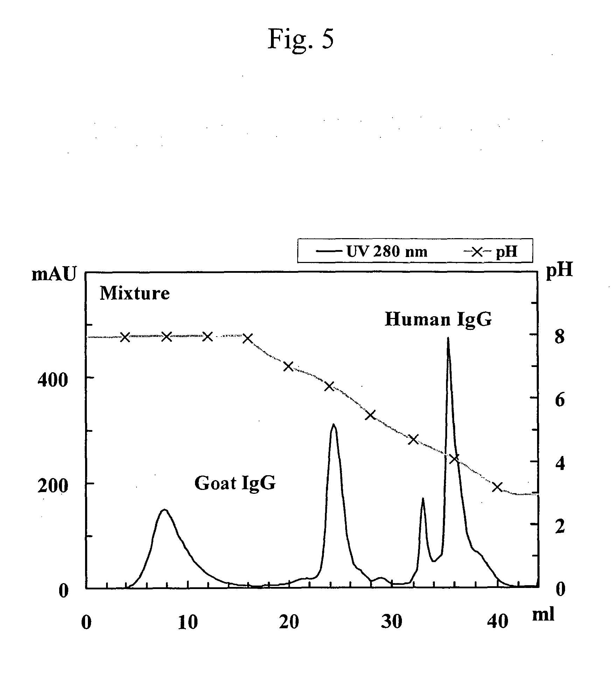

The following 3 samples were separately applied to 3 protein A columns (POROS A/20; 4.6 mm ID x 5 cm; Applied Biosystems) that had been equilibrated with a buffer (pH 8.0) containing 0.10 M disodium phosphate, 0.10 M sodium acetate, 0.10 M glycine, and 0.15 M sodium chloride (linear flow rate: 90 cm/h). The 3 samples were as follows: (i) 1 ml of solution containing human polyclonal antibodies; (ii) 1 ml of solution containing goat polyclonal antibodies; and (iii) 2 ml of mixed solution containing equivalent amounts of human polyclonal antibodies and goat polyclonal antibodies. These sample solutions were separately applied to the columns. After the completion of application, the columns were washed with 5 column volumes of buffer for equilibration (linear flow rate: 90 cm/h). Subsequently, human polyclonal antibodies and goat polyclonal antibodies were eluted via pH gradient elution (gradient volume: 30 column volumes of buffer were used to bring the pH level from 8.0 to 2.5) in which the pH level was gradually lowered by gradually adding a buffer (pH 2.5) containing 0.10 M sodium acetate, 0.10 M glycine, and 0.15 M sodium chloride to a buffer containing 0.10 M disodium phosphate, 0.10 M sodium acetate, 0.10 M glycine, and 0.15 M sodium chloride (linear flow rate: 90 cm/h). The elution curves obtained via the aforementioned chromatography techniques are shown in Figs. 4 and 5.

As shown in Figs. 4 and 5, the rate of elution of goat polyclonal antibodies was faster than that of human polyclonal antibodies in the case of pH gradient elution. As shown in Fig. 5, human polyclonal antibodies were separated from goat polyclonal antibodies via chromatography of a mixture containing human and goat polyclonal antibodies.

### Example 5: Separation and purification of human polyclonal antibodies from sheep polyclonal antibodies

Human polyclonal antibodies (3 mg; trade name: Human IgG from Serum; Cat No. 14506; Sigma) and sheep polyclonal antibodies (3 mg; trade name: Sheep IgG from Serum; Cat No. I5131; Sigma) were each separately dissolved in 3 ml of buffer (pH 8.0) containing 0.10 M disodium phosphate, 0.10 M sodium acetate, 0.10 M glycine, and 0.15 M sodium chloride. Thereafter, the resulting solutions were allowed to pass through a 0.2-µm filter and then separately designated as the solution of human polyclonal antibodies and the solution of sheep polyclonal antibodies.

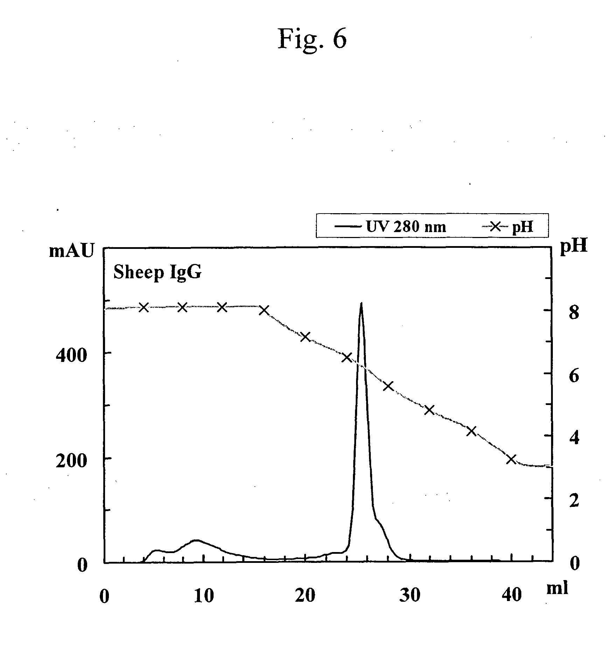

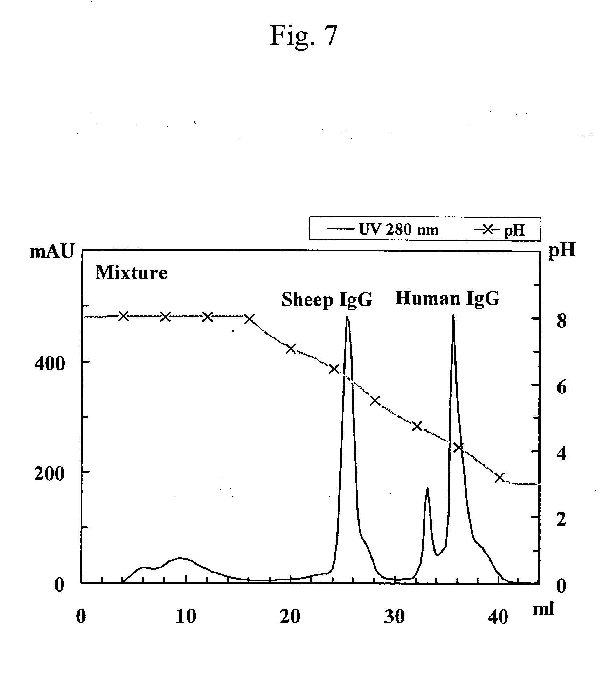

The following 3 samples were separately applied to 3 protein A columns (POROS A/20; 4.6 mm ID x 5 cm; Applied Biosystems) that had been equilibrated with a buffer (pH 8.0) containing 0.10 M disodium phosphate, 0.10 M sodium acetate, 0.10 M glycine, and 0.15 M sodium chloride (linear flow rate: 90 cm/h). The 3 samples were as follows: (i) 1 ml of solution containing human polyclonal antibodies; (ii) 1 ml of solution containing sheep polyclonal antibodies; and (iii) 2 ml of mixed solution containing equivalent amounts of human polyclonal antibodies and sheep polyclonal antibodies. These sample solutions were separately applied to the columns. After the completion of application, the columns were washed with 5 column volumes of buffer for equilibration (linear flow rate: 90 cm/h). Subsequently, human polyclonal antibodies and sheep polyclonal antibodies were eluted via pH gradient elution (gradient volume: 30 column volumes of buffer were used to bring the pH level from 8.0 to 2.5) in which the pH level was gradually lowered by gradually adding a buffer (pH 2.5) containing 0.10 M sodium acetate, 0.10 M glycine, and 0.15 M sodium chloride to a buffer containing 0.10 M disodium phosphate, 0.10 M sodium acetate, 0.10 M glycine, and 0.15 M sodium chloride (linear flow rate: 90 cm/h). The elution curves obtained via the aforementioned chromatography techniques are shown in Figs. 6 and 7.

As shown in Figs. 6 and 7, the rate of elution of sheep polyclonal antibodies was faster than that of human polyclonal antibodies in the case of pH gradient elution. As shown in Fig. 7, human polyclonal antibodies were separated from sheep polyclonal antibodies via chromatography of a mixture containing human and sheep polyclonal antibodies.

### Industrial Applicability

(1) According to the purification sequence of anion exchange chromatography followed by cation exchange chromatography according to the present invention, a step of dilution that has been essential for the conventional purification sequence of cation exchange chromatography followed by anion exchange chromatography was significantly simplified. This resolved problems such as (i) necessity of a solution for dilution, (ii) necessity of an enlarged apparatus due to an increased volume resulting from dilution, and (iii) prolonged operating time. (2) According to the present invention, a method for isolating human antibodies from a mixture containing artiodactyl antibodies and human antibodies was established, which had represented an unresolved issue in the past.

As described in Example 3, purified antibodies that can be safely utilized as pharmaceuticals can be obtained by additionally carrying out the step of virus inactivation or the step of virus removal during the process of purification.

All publications cited herein are incorporated herein in their entirety. A person skilled in the art would easily understand that various modifications and changes of the present invention are feasible within the technical idea and the scope of the invention as disclosed in the attached claims. The present invention is intended to include such modifications and changes.

## Claims

1. A method for separating and purifying antibodies from an antibody-containing mixture comprising at least the following steps of:
(1) purification via anion exchange chromatography; and
(2) purification via cation exchange chromatography following step (1).

2. A method for separating and purifying antibodies from an antibody-containing mixture comprising at least the following steps of:
(1) purification via protein A affinity chromatography;
(2) purification via anion exchange chromatography following step (1); and
(3) purification via cation exchange chromatography following step (2).

3. A method for separating and purifying antibodies from an antibody-containing mixture comprising at least the following steps of:
(1) purification via protein A affinity chromatography;
(2) purification via anion exchange chromatography following step (1);
(3) purification via cation exchange chromatography following step (2); and
(4) purification via hydrophobic chromatography following step (3).

4. A method for separating and purifying antibodies from an antibody-containing mixture comprising at least the following steps of:
(1) applying an antibody-containing mixture to a protein A column equilibrated with a buffer containing sodium phosphate and sodium chloride to bring the mixture into contact with the protein A column;
(2) washing the protein A column with the buffer used for equilibration in step (1) following step (1);
(3) eluting the antibodies with a sodium citrate buffer following step (2);
(4) adjusting the pH of an eluate to 4.0 to 8.5 with a sodium phosphate buffer following step (3);
(5) adjusting the pH of the eluate of step (4) to 6.0 to 8.5 with a Tris-HCl buffer following step (4);
(6) applying the eluate of step (5) to an anion exchange column equilibrated with a Tris-HCl buffer to bring the eluate into contact with the anion exchange column following step (5);
(7) eluting the antibodies from the anion exchange column with the buffer used for equilibration in step (6) following step (6);
(8) adjusting the pH of the eluate of step (7) to 4.0 to 7.0 with acetic acid or citric acid following step (7);
(9) applying the eluate of step (8) to a cation exchange column equilibrated with a sodium acetate buffer or a sodium phosphate-citrate buffer to bring the eluate into contact with the cation exchange column following step (8);
(10) washing the cation exchange column with the buffer used for equilibration in step (9) following step (9); and
(11) eluting the antibodies from the cation exchange column with a sodium acetate buffer or a sodium phosphate-citrate buffer and a buffer containing sodium chloride following step (10).

5. A method for separating and purifying antibodies from an antibody-containing mixture, which further comprises the steps of:
(12) applying ammonium sulfate to the eluate of step (11) of claim 4 to adjust the pH of the eluate to 6.0 to 8.0 with an aqueous solution of sodium hydroxide following step (11) of claim 4;
(13) applying the eluate of step (12) to a hydrophobic column equilibrated with a buffer containing ammonium sulfate and sodium phosphate to bring the eluate into contact with the hydrophobic column following step (12);
(14) washing the hydrophobic column with the buffer used for equilibration in step (13) following step (13); and
(15) eluting the antibodies from the hydrophobic column by lowering the ammonium sulfate concentration in the buffer of step (13) following step (14).

6. The method according to any one of claims 1 to 5, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

7. The method according to claim 6, wherein the step of virus inactivation is carried out following the step of purification via protein A chromatography.

8. The method according to claim 7, wherein the step of virus inactivation is carried out following step (3) of claim 4 by adjusting the pH of the eluate obtained in step (3) to 4 or lower and allowing the eluate to stand for 30 minutes or longer.

9. The method according to any one of claims 6 to 8, wherein the step of virus removal via filtration with the use of a virus removal filter and/or the step of aseptic filtration are carried out in the final step of the method for separating and purifying antibodies from an antibody-containing mixture.

10. The method according to any one of claims 4 to 9, wherein a Tris-HCl buffer is used instead of the sodium phosphate buffer of step (4) of claim 4.

11. The method according to any one of claims 4 to 9, wherein the following step is carried out instead of step (4) and step (5) of claim 4:
a step of adjusting the pH of an eluate to 6.0 to 8.5 with a Tris-HCl buffer following step (3).

12. A method for separating and purifying antibodies from an antibody-containing mixture comprising at least the following steps of:
(1) applying an antibody-containing mixture to a protein A column equilibrated with a buffer containing 10 mM to 100 mM sodium phosphate and 0 M to 4.0 M sodium chloride to bring the mixture into contact with the protein A column;
(2) washing the protein A column with the buffer used for equilibration in step (1) following step (1);
(3) eluting the antibodies with a 10 mM to 100 mM sodium citrate buffer following step (2);
(4) adjusting the pH of an eluate to 4.0 to 8.5 with a 10 mM to 200 mM sodium phosphate buffer following step (3);
(5) adjusting the pH of the eluate of step (4) to 6.0 to 8.5 with a 0.5 M to 1.5 M Tris-HCl buffer following step (4);
(6) applying the eluate of step (5) to an anion exchange column equilibrated with a 10 mM to 20 mM Tris-HCl buffer to bring the eluate into contact with the anion exchange column following step (5);
(7) eluting the antibodies from the anion exchange column with the buffer used for equilibration in step (6) following step (6);
(8) adjusting the pH of the eluate of step (7) to 4.0 to 7.0 with 0.5 M to 1.5 M acetic acid or citric acid following step (7);
(9) applying the eluate of step (8) to a cation exchange column equilibrated with a 10 mM to 50 mM sodium acetate buffer or a 10 mM to 50 mM sodium phosphate-10 mM to 50 mM citrate buffer to bring the eluate into contact with the cation exchange column following step (8);
(10) washing the cation exchange column with the buffer used for equilibration in step (9) following step (9); and
(11) eluting the antibodies from the cation exchange column with a 10 mM to 50 mM sodium acetate or 10 mM to 50 mM sodium phosphate-10 mM to 50 mM citrate buffer and a buffer containing 0.1 M to 1.0 M sodium chloride following step (10).

13. A method for separating and purifying antibodies from an antibody-containing mixture, which further comprises the steps of:
(12) applying ammonium sulfate to the eluate of step (11) of claim 12 to bring the ammonium sulfate concentration thereof to 0.8 M to 1.2 M and adjusting the pH thereof to 6.0 to 8.0 with an aqueous solution of 1.0 M to 10.0 M sodium hydroxide following step (11) of claim 12;
(13) applying the eluate of step (12) to a hydrophobic column equilibrated with a buffer containing 0.8 M to 1.2 M ammonium sulfate and 10 mM to 100 mM sodium phosphate to bring the eluate into contact with the hydrophobic column following step (12);
(14) washing the hydrophobic column with the buffer used for equilibration in step (13) following step (13); and
(15) eluting the antibodies from the hydrophobic column by lowering the ammonium sulfate concentration in the buffer of step (13) following step (14).

14. The method according to claim 12 or 13, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

15. The method according to claim 14, wherein the step of virus inactivation is carried out following step (3) of claim 12 by adjusting the pH of the eluate obtained in step (3) to 4 or lower and allowing the eluate to stand for 30 minutes or longer.

16. The method according to claim 14 or 15, wherein the step of virus removal via filtration with the use of a virus removal filter and/or the step of aseptic filtration are carried out following step (11) of claim 12.

17. The method according to claim 14 or 15, wherein the step of virus removal via filtration with the use of a virus removal filter and/or the step of aseptic filtration are carried out following step (15) of claim 13.

18. The method according to any one of claims 12 to 17, wherein a 0.5 M to 1.5 M Tris-HCl buffer is used instead of the sodium phosphate buffer of step (4) of claim 12.

19. The method according to any one of claims 12 to 17, wherein the following step is carried out instead of step (4) and step (5) of claim 12:
a step of adjusting the pH of an eluate to 6.0 to 8.5 with a 0.5 M to 1.5 M Tris-HCl buffer following step (3).

20. A method for separating and purifying antibodies from an antibody-containing mixture comprising at least the following steps of:
(1) applying an antibody-containing mixture to a protein A column equilibrated with a buffer containing 10 mM to 20 mM sodium phosphate and 0.15 M sodium chloride to bring the mixture into contact with the protein A column;
(2) washing the protein A column with the buffer used for equilibration in step (1) following step (1);
(3) eluting the antibodies with a 10 mM to 20 mM sodium citrate buffer following step (2);
(4) adjusting the pH of an eluate to 4.0 to 8.5 with a 10 mM to 100 mM sodium phosphate buffer following step (3);
(5) adjusting the pH of the eluate of step (4) to 6.0 to 8.5 with a 1.0 M to 1.5 M Tris-HCl buffer following step (4);
(6) applying the eluate of step (5) to an anion exchange column equilibrated with a 10 mM Tris-HCl buffer to bring the eluate into contact with the anion exchange column following step (5);
(7) eluting the antibodies from the anion exchange column with the buffer used for equilibration in step (6) following step (6);
(8) adjusting the pH of the eluate of step (7) to 4.0 to 7.0 with 1 M acetic acid or citric acid following step (7);
(9) applying the eluate of step (8) to a cation exchange column equilibrated with a 20 mM sodium acetate buffer or a 20 mM sodium phosphate-10 mM citrate buffer to bring the eluate into contact with the cation exchange column following step (8);
(10) washing the cation exchange column with the buffer used for equilibration in step (9) following step (9); and
(11) eluting the antibodies from the cation exchange column with a 20 mM sodium acetate or 20 mM sodium phosphate-10 mM citrate buffer and a buffer containing 0.3 M sodium chloride following step (10).

21. A method for separating and purifying antibodies from an antibody-containing mixture, which further comprises the steps of:
(12) applying ammonium sulfate to the eluate of step (11) of claim 20 to bring the ammonium sulfate concentration thereof to 1.0 M and adjusting the pH thereof to 6.0 to 8.0 with an aqueous solution of 10.0 M sodium hydroxide following step (11) of claim 20;
(13) applying the eluate of step (12) to a hydrophobic column equilibrated with a buffer containing 1.0 M ammonium sulfate and 10 mM sodium phosphate to bring the eluate into contact with the hydrophobic column following step (12);
(14) washing the hydrophobic column with the buffer used for equilibration in step (13) following step (13); and
(15) eluting the antibodies from the hydrophobic column by lowering the ammonium sulfate concentration in the buffer of step (13) following step (14).

22. The method according to claim 20 or 21, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

23. The method according to claim 22, wherein the step of virus inactivation is carried out following step (3) of claim 20 by adjusting the pH of the eluate obtained in step (3) to 4 or lower and allowing the eluate to stand for 30 minutes or longer.

24. The method according to claim 22 or 23, wherein the step of virus removal via filtration with the use of a virus removal filter and/or the step of aseptic filtration are carried out following step (11) of claim 20.

25. The method according to claim 22 or 23, wherein the step of virus removal via filtration with the use of a virus removal filter and/or the step of aseptic filtration are carried out following step (15) of claim 21.

26. The method according to any one of claims 20 to 25, wherein a 1.0 M Tris-HCl buffer is used instead of the sodium phosphate buffer of step (4) of claim 20.

27. The method according to any one of claims 20 to 25, wherein the following step is carried out instead of step (4) and step (5) of claim 20:
a step of adjusting the pH of an eluate to 6.0 to 8.5 with a 1.0 M Tris-HCl buffer following step (3).

28. A method for separating and purifying antibodies from an antibody-containing mixture comprising at least the following steps of:
(1) applying an antibody-containing mixture to a protein A column equilibrated with a buffer containing 10 mM to 20 mM sodium phosphate and 0.15 M sodium chloride to bring the mixture into contact with the protein A column;
(2) washing the protein A column with the buffer used for equilibration in step (1) following step (1);
(3) eluting the antibodies with a 10 mM to 20 mM sodium citrate buffer following step (2);
(4) adjusting the pH of an eluate to 7.0 with a 10 mM to 100 mM sodium phosphate buffer following step (3);
(5) adjusting the pH of the eluate of step (4) to 8.0 with a 1.0 M to 1.5 M Tris-HCl buffer following step (4);
(6) applying the eluate of step (5) to an anion exchange column equilibrated with a 10 mM Tris-HCl buffer to bring the eluate into contact with the anion exchange column following step (5);
(7) eluting the antibodies from the anion exchange column with the buffer used for equilibration in step (6) following step (6);
(8) adjusting the pH of the eluate of step (7) to 5.0 with 1.0 M acetic acid or citric acid following step (7);
(9) applying the eluate of step (8) to a cation exchange column equilibrated with a 20 mM sodium acetate buffer or a 20 mM sodium phosphate-10 mM citrate buffer to bring the eluate into contact with the cation exchange column following step (8);
(10) washing the cation exchange column with the buffer used for equilibration in step (9) following step (9); and
(11) eluting the antibodies from the cation exchange column with a 20 mM sodium acetate or 20 mM sodium phosphate-10 mM citrate buffer and a buffer containing 0.3 M sodium chloride following step (10).

29. A method for separating and purifying antibodies from an antibody-containing mixture, which further comprises the steps of:
(12) adding ammonium sulfate to the eluate of step (11) of claim 28 to bring the ammonium sulfate concentration thereof to 1.0 M and adjusting the pH thereof to 7.0 with an aqueous solution of 10.0 M sodium hydroxide following step (11) of claim 28;
(13) applying the eluate of step (12) to a hydrophobic column equilibrated with a buffer containing 1.0 M ammonium sulfate and 10 mM sodium phosphate to bring the eluate into contact with the hydrophobic column following step (12);
(14) washing the hydrophobic column with the buffer used for equilibration in step (13) following step (13); and
(15) eluting the antibodies from the hydrophobic column by lowering the ammonium sulfate concentration in the buffer of step (13) following step (14).

30. The method according to claim 28 or 29, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

31. The method according to claim 30, wherein the step of virus inactivation is carried out following step (3) of claim 28 by adjusting the pH of the eluate obtained in step (3) to 4 or lower and allowing the eluate to stand for 30 minutes or longer.

32. The method according to claim 30 or 31, wherein the step of virus removal via filtration with the use of a virus removal filter and/or the step of aseptic filtration are carried out following step (11) of claim 28.

33. The method according to claim 30 or 31, wherein the step of virus removal via filtration with the use of a virus removal filter and/or the step of aseptic filtration are carried out following step (15) of claim 29.

34. The method according to any one of claims 28 to 33, wherein a 1.0 M Tris-HCl buffer is used instead of the sodium phosphate buffer of step (4) of claim 28.

35. The method according to any one of claims 28 to 33, wherein the following step is carried out instead of step (4) and step (5) of claim 28:
a step of adjusting the pH of an eluate to 8.0 with a 1.0 M Tris-HCl buffer following step (3).

36. A method for separating and purifying antibodies from an antibody-containing mixture comprising at least the following steps of:
(1) applying an antibody-containing mixture to a protein A column equilibrated with a buffer of pH 6.0 to 8.5 to bring the mixture into contact with the protein A column;
(2) washing the protein A column with the buffer of pH 6.0 to 8.5 following step (1);
(3) eluting the antibodies with a buffer of pH 2.7 to 4.0 following step (2);
(4) adjusting the pH of an eluate to 4.0 to 8.5 following step (3);
(5) adjusting the pH of the eluate of step (4) to 6.0 to 8.5 following step (4);
(6) applying the eluate of step (5) to an anion exchange column equilibrated with a buffer of pH 6.0 to 8.5 to bring the eluate into contact with the anion exchange column following step (5);
(7) eluting the antibodies from the anion exchange column with the buffer of pH 6.0 to 8.5 following step (6);
(8) adjusting the pH of the eluate of step (7) to 4.0 to 7.0 following step (7);
(9) applying the eluate of step (8) to a cation exchange column equilibrated with a buffer of pH 4.0 to 7.0 to bring the eluate into contact with the cation exchange column following step (8);
(10) washing the cation exchange column with a buffer of pH 4.0 to 7.0 following step (9); and
(11) eluting the antibodies from the cation exchange column with a buffer of pH 4.0 to 7.0 with a salt concentration of 0.1 M to 1.0 M following step (10).

37. A method for separating and purifying antibodies from an antibody-containing mixture, which further comprises the steps of:
(12) applying salt to the eluate of step (11) of claim 36 to bring the salt concentration thereof to 0.8 M to 1.2 M and adjusting the pH thereof to 6.0 to 8.0;
(13) applying the eluate of step (12) to a hydrophobic column equilibrated with a buffer of pH 6.0 to 8.0 containing the salt of step (12) at 0.8 M to 1.2 M to bring the eluate into contact with the hydrophobic column;
(14) washing the hydrophobic column with the buffer of pH 6.0 to 8.0 containing the salt of step (12) at 0.8 M to 1.2 M; and
(15) eluting the antibodies from the hydrophobic column by lowering the salt concentration in the buffer of step (13).

38. The method according to claim 36 or 37, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

39. The method according to claim 38, wherein the step of virus inactivation is carried out following step (3) of claim 36 by adjusting the pH of the eluate obtained in step (3) to 4 or lower and allowing the eluate to stand for 30 minutes or longer.

40. The method according to claim 38 or 39, wherein the step of virus removal via filtration with the use of a virus removal filter and/or the step of aseptic filtration are carried out following step (11) of claim 36.

41. The method according to claim 38 or 39, wherein the step of virus removal via filtration with the use of a virus removal filter and/or the step of aseptic filtration are carried out following step (15) of claim 37.

42. The method according to claim 36, wherein the buffer of pH 6.0 to 8.5 used in step (2) is identical to the buffer used in step (1), the buffer of pH 6.0 to 8.5 used in step (7) is identical to the buffer used in step (6), and the buffer of pH 4.0 to 7.0 used in step (10) is identical to the buffer used in step (9).

43. The method according to any one of claims 37 to 42, wherein the buffer of pH 6.0 to 8.0 containing the salt of step (12) used in step (14) of claim 37 at 0.8 M to 1.2 M is identical to the buffer used in step (13).

44. The method according to any one of claims 36 to 43, wherein the salt used in step (11) of claim 36 is sodium chloride.

45. The method according to any one of claims 36 to 44, wherein the salt used in step (12) of claim 37 is ammonium sulfate.

46. The method according to any one of claims 36 to 45, wherein the salt concentration of the buffer used in step (1) of claim 36 is 4.0 M or lower.

47. The method according to claim 46, wherein the salt is sodium chloride.

48. A method for separating and purifying antibodies from an antibody-containing mixture comprising at least the following steps of:
(1) applying an antibody-containing mixture to an anion exchange column equilibrated with a buffer of pH 6.0 to 8.5 to bring the mixture into contact with the anion exchange column;
(2) eluting the antibodies from the anion exchange column with a buffer of pH 6.0 to 8.5 following step (1);
(3) adjusting the pH of the eluate of step (2) to 4.0 to 7.0 following step (2);
(4) applying the eluate of step (3) to a cation exchange column equilibrated with a buffer of pH 4.0 to 7.0 to bring the eluate into contact with the cation exchange column following step (3);
(5) washing the cation exchange column with a buffer of pH 4.0 to 7.0 following step (4); and
(6) eluting the antibodies from the cation exchange column with a buffer of pH 4.0 to 7.0 with a salt concentration of 0.1 M to 1.0 M following step (5).

49. The method according to claim 48, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

50. The method according to claim 49, wherein the step of virus inactivation is carried out before step (1) of claim 48 by adjusting the pH of the antibody-containing solution to 4 or lower and allowing the eluate to stand for 30 minutes or longer.

51. The method according to claim 49 or 50, wherein the step of virus removal via filtration with the use of a virus removal filter and/or the step of aseptic filtration are carried out following step (6) of claim 48.

52. The method according to any one of claims 48 to 51, wherein the buffer of pH 6.0 to 8.5 used in step (2) of claim 48 is identical to the buffer used in step (1) and the buffer of pH 4.0 to 7.0 used in step (5) of claim 48 is identical to the buffer used in step (4).

53. The method according to any one of claims 48 to 52, wherein the salt used in step (6) of claim 48 is sodium chloride.

54. The method according to any one of claims 1 to 53, wherein the antibodies are human antibodies.

55. The method according to any one of claims 1 to 54, wherein the antibodies are monoclonal antibodies.

56. The method according to any one of claims 1 to 55, wherein the antibodies are IgG antibodies.

57. The method according to claim 56, wherein the antibodies are IgG1, IgG2, or IgG4 antibodies.

58. The method according to any one of claims 54 to 57, wherein the antibodies have an amino acid sequence derived from the amino acid sequence of the heavy chain constant region by deletion, substitution, or addition of at least one amino acid residue in the naturally occurring heavy chain constant region.

59. The method according to any one of claims 54 to 58, wherein the antibodies are covalently or coordinately bound to other compounds.

60. A method for separating human antibodies from ungulate antibodies via protein A affinity chromatography from a mixture containing human antibodies and ungulate antibodies.

61. The method according to claim 60, wherein the ungulate is selected from the group consisting of a bovine, a goat, and a sheep.

62. The method according to claim 60 or 61, wherein separation is carried out via pH gradient elution.

63. The method according to claim 60 or 61, wherein separation is carried out via pH stepwise elution.

64. A method for isolating human antibodies from a mixture containing human antibodies and ungulate antibodies comprising at least the following steps of:
(1) applying a mixture containing human antibodies and ungulate antibodies to a protein A column equilibrated with a buffer containing disodium phosphate, sodium acetate, glycine, and sodium chloride to bring the mixture into contact with the protein A column;
(2) washing the protein A column with the buffer of step (1) following step (1); and
(3) eluting the human antibodies from the protein A column by lowering the pH of the buffer of step (1) following step (2).

65. The method according to claim 64, wherein the ungulate is selected from the group consisting of a bovine, a goat, and a sheep.

66. The method according to claim 64 or 65, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

67. The method according to claim 66, wherein the step of virus inactivation is carried out following step (3) of claim 64 by adjusting the pH of the eluate obtained in step (3) to 4 or lower and allowing the eluate to stand for 30 minutes or longer.

68. The method according to claim 66 or 67, wherein the step of virus removal via filtration with the use of a virus removal filter and/or the step of aseptic filtration are carried out so as to comprise the final step of the method for isolating human antibodies from a mixture containing human antibodies and ungulate antibodies.

69. The method according to any one of claims 64 to 68, wherein the pH level is lowered in a gradient manner.

70. The method according to any one of claims 64 to 68, wherein the pH level is lowered stepwise.

71. A method for isolating human antibodies from a mixture containing human antibodies and ungulate antibodies comprising at least the following steps of:
(1) applying a mixture containing human antibodies and ungulate antibodies to a protein A column equilibrated with a buffer containing 0.05 M to 0.15 M disodium phosphate, 0.05 M to 0.15 M sodium acetate, 0.05 M to 0.15 M glycine, and 0.05 M to 0.20 M sodium chloride to bring the mixture into contact with the protein A column;
(2) washing the protein A column with the buffer of step (1) following step (1); and
(3) eluting the human antibodies from the protein A column by lowering the pH of the buffer of step (1) following step (2).

72. The method according to claim 71, wherein the ungulate is selected from the group consisting of a bovine, a goat, and a sheep.

73. The method according to claim 71 or 72, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

74. The method according to claim 73, wherein the step of virus inactivation is carried out following step (3) of claim 71 by adjusting the pH of the eluate obtained in step (3) to 4 or lower and allowing the eluate to stand for 30 minutes or longer.

75. The method according to claim 73 or 74, wherein the step of virus removal via filtration with the use of a virus removal filter and/or the step of aseptic filtration are carried out so as to comprise the final step of the method for isolating human antibodies from a mixture containing human antibodies and ungulate antibodies.

76. The method according to any one of claims 71 to 75, wherein the pH level is lowered in a gradient manner.

77. The method according to any one of claims 71 to 75, wherein the pH level is lowered stepwise.

78. A method for isolating human antibodies from a mixture containing human antibodies and ungulate antibodies comprising at least the following steps of:
(1) applying a mixture containing human antibodies and ungulate antibodies to a protein A column equilibrated with a buffer containing 0.10 M disodium phosphate, 0.10 sodium acetate, 0.10 M glycine, and 0.15 M sodium chloride to bring the mixture into contact with the protein A column;
(2) washing the protein A column with the buffer of step (1) following step (1); and
(3) eluting the human antibodies from the protein A column by lowering the pH of the buffer of step (1) following step (2).

79. The method according to claim 78, wherein the ungulate is selected from the group consisting of a bovine, a goat, and a sheep.

80. The method according to claim 78 or 79, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

81. The method according to claim 80, wherein the step of virus inactivation is carried out following step (3) of claim 78 by adjusting the pH of the eluate obtained in step (3) to 4 or lower and allowing the eluate to stand for 30 minutes or longer.

82. The method according to claim 80 or 81, wherein the step of virus removal via filtration with the use of a virus removal filter and/or the step of aseptic filtration are carried out so as to comprise the final step of the method for isolating human antibodies from a mixture containing human antibodies and ungulate antibodies.

83. The method according to any one of claims 78 to 82, wherein the pH level is lowered in a gradient manner.

84. The method according to any one of claims 78 to 82, wherein the pH level is lowered stepwise.

85. A method for isolating human antibodies from a mixture containing human antibodies and ungulate antibodies comprising at least the following steps of:
(1) applying a mixture containing human antibodies and ungulate antibodies to a protein A column equilibrated with a buffer of pH 7.5 to 8.5 to bring the mixture into contact with the protein A column;
(2) washing the protein A column with a buffer of pH 7.5 to 8.5; and
(3) eluting the human antibodies from the protein A column by lowering the pH.

86. The method according to claim 85, wherein the ungulate is selected from the group consisting of a bovine, a goat, and a sheep.

87. The method according to claim 85 or 86, which further comprises one or more of the steps of: virus inactivation by allowing the antibody-containing solution to stand under acidic conditions; virus removal from the antibody-containing solution with the use of a virus removal filter; and aseptic filtration of the antibody-containing solution.

88. The method according to claim 87, wherein the step of virus inactivation is carried out following step (3) of claim 85 by adjusting the pH of the eluate obtained in step (3) to 4 or lower and allowing the eluate to stand for 30 minutes or longer.

89. The method according to claim 87 or 88, wherein the step of virus removal via filtration with the use of a virus removal filter and/or the step of aseptic filtration are carried out so as to comprise the final step of the method for isolating human antibodies from a mixture containing human antibodies and ungulate antibodies.

90. The method according to any one of claims 85 to 89, wherein the pH level is lowered in a gradient manner.

91. The method according to any one of claims 85 to 89, wherein the pH level is lowered stepwise.

92. The method according to any one of claims 85 to 91, wherein the buffer of pH 7.5 to 8.5 used in step (2) of claim 85 is identical to the buffer used in step (1).

93. The method according to any one of claims 85 to 92, wherein a salt concentration of the buffer of pH 7.5 to 8.5 used in step (1) of claim 85 is 0.05 M to 0.20 M.

94. The method according to claim 93, wherein the salt is sodium chloride.

95. The method according to any one of claims 60 to 94, wherein the human antibodies are human polyclonal antibodies.

96. The method according to any one of claims 60 to 95, wherein the ungulate antibodies are selected from the group consisting of bovine polyclonal antibodies, goat polyclonal antibodies, and sheep polyclonal antibodies.

97. The method according to any one of claims 60 to 96, wherein the human antibodies are IgG antibodies.

98. The method according to claim 97, wherein the human antibodies are IgG1 antibodies, IgG2 antibodies, or IgG4 antibodies.
